# EUROPEAN PATENT APPLICATION

(11) **EP 1 873 251 A1**
(43) Date of publication of application: **02.01.2008**
(21) Application number: 06013507.6
(22) Date of filing: 29.06.2006
(51) Int. Cl.: C12N 15/70, C12N 15/79

(54) **Expression vector(s) for enhanced expression of a protein of interest in eukaryotic or prokaryotic host cells**

(71) Applicant: CHEMOTHERAPEUTISCHES FORSCHUNGSINSTITUT GEORG-SPEYER-HAUS, D-60596 Frankfurt (DE)
(72) Inventor: Wels, Winfried, Dr., 60599 Frankfurt (DE); Dälken, Benjamin, Dr., 60433 Frankfurt (DE)
(74) Representative: Graf von Stosch, Andreas

(57) **Abstract**

The present invention refers to an expression vector (A) for enhanced expression of a protein of interest encoding a fusion protein comprising at least a secretory signal sequence (A1); a solubilizing agent (A2); a proteolytic cleavage site (A3); and a protein of interest (A4), optionally a tag for purification of the protein of interest (A5) and optionally a heterologously expressed protease (A6), capable of cleaving the proteolytic cleavage site (A3) subsequent to or during secretion of the fusion protein. Alternatively, the protease (A6) may be endogenously expressed in the host cell, may be provided to the host cell using a second expression vector (B) and/or may be added extracellularly. The present invention furthermore provides methods for using the inventive expression vectors (A) and, optionally, (*B*), *in vitro* and *in vivo,* and kits, comprising these expression vectors and/or cells comprising same.

## Description

The present invention refers to an expression vector (A) for enhanced expression of a protein of interest encoding a fusion protein comprising at least a secretory signal sequence (A1); a solubilizing agent (A2); a proteolytic cleavage site (A3); and a protein of interest (A4), optionally a tag for purification of the protein of interest (A5) and optionally a heterologously expressed protease (A6), capable of cleaving the proteolytic cleavage site (A3) subsequent to or during secretion of the fusion protein. Alternatively, the protease (A6) may be endogenously expressed in the host cell, may be provided to the host cell using a second expression vector (B) and/or may be added extracellularly. The present invention furthermore provides methods for using the inventive expression vectors (A) and, optionally, (B), *in vitro* and *in vivo,* and kits, comprising these expression vectors and/or cells comprising same.

Heterologous expression and purification of recombinant proteins *in vitro* and *in vivo* represent routine applications of modern molecular biology. Generally, heterologous expression of recombinant proteins in prokaryotic or eukaryotic host cells is easy to handle and typically results in average expression levels of the resultant recombinant protein, usually with higher protein yields in prokaryotic host cells than in eukaryotic host cells. However, adaptation and, if necessary, increase of expression levels still is challenging for a skilled person and is often closely related to specific requirements of the host cell system used.

Expression of recombinant proteins is typically carried out in prokaryotic host cells or, alternatively, in eukaryotic host cells. Adaptation of these host cells may lead to elevated expression levels of a protein of interest. However, the protein of interest usually accumulates by forming insoluble aggregates called inclusion bodies, if high expression levels are intended to be obtained in these host cells. Inclusion bodies typically contain the protein of interest in a misfolded and biologically inactive form, e.g. as partially folded protein chains. Inclusion body formation is considered to occur via non-specific intermolecular association of hydrophobic surfaces in folding intermediates (Fahnert et al., Adv Biochem Eng Biotechnol 2004, 89:93-142; Gribskov and Burgess, Gene 1983, 26:109-18; Ho and Middelberg, Biotechnol Bioeng, 2004, 87:584-92). Various purification protocols have already been established in order to convert the isolated inclusion bodies into soluble proteins. Thereby, most efforts have been directed to refold purified inclusion body proteins by chemical denaturation followed by refolding procedures. However, this strategy needs to be modified depending on the particular protein species involved and provides unpredictable and unsatisfying results for the final soluble protein yield (Garcia-Fruitos *et al.,* Microb Cell Fact 2005, 4:27). This finding may be due to the fact that chemical denaturation and a subsequent refolding step does not ensure correct folding of the protein of interest and the protein of interest may thus remain in solution in a misfolded and biologically inactive form. Furthermore, purification protocols intended to convert inclusion bodies into soluble (and biologically and/or enzymatically active) proteins are typically time consuming. Accordingly, avoidance of inclusion body formation during expression of proteins in host cells is highly desired.

Present strategies to avoid formation of inclusion bodies include e.g. increase of protein solubility. Increase of protein solubility according to the art is achieved either by modification of the process of protein translation in the host cell or involves protein engineering and site-directed mutagenesis techniques, e.g. by substituting hydrophobic amino acid residues located at the protein surface.

Another attempt to increase protein solubility of (heterologously) expressed proteins of interest and prevention of inclusion body formation is to express the protein of interest as fusion protein with a highly soluble protein component as solubilizing agent (Nygren et al., Trends Biotechnol 1994, 12:184-8; Uhlen et al., Curr Opin Biotechnol 1992, 3:363-9).

Protein components that are employed as solubilizing agents include e.g. glutathion-S-transferase (GST) (Tudyka and Skerra, Protein Sci 1997, 6:2180-7), thioredoxin (TRX) (LaVallie et al., Biotechnology (NY) 1993, 11:187-93), ubiquitin (Power et al., J Biol Chem 1990, 265:1419-24), protein A (Samuelsson et al., Biochemistry 1994, 33:4207-11), DsbA (Zhang et al., Protein Expr. Purif. 1998, 12:159-65), domain 1 of IF2 protein of *E*. *coli* (Sorensen et al., Protein Expr Purif 2003, 32:252-9) and maltose-binding protein (MBP) (di Guan et al., Gene 1988, 67:21-30; Kapust and Waugh, Protein Sci 1999, 8:1668-74).

Among the above mentioned solubilizing agents, maltose-binding protein (MBP) from *E*. *coli* is preferably used as fusion partner. MBP is a natural periplasmic protein of *E. coli* and part of a large class of proteins that aid in uptake of small molecules and also act as sensors for signaling through the chemotaxis system (Ehrmann et al., Mol Microbiol 1998, 29:685-94). MBP is expressed in its physiological compartment, the bacterial periplasm, and is also available by expression in the cytoplasm of *E. coli* at high yields. It has been shown in the art that solubility and stability of several proteins expressed in prokaryotic host cells such as *E. coli* can be enhanced by fusion with MBP (Hayhurst A., Protein Expr Purif 2000, 18:1-10; Jacquet et al., Protein Expr Purif 1999, 17:392-400; Pryor and Leiting, Protein Expr Purif 1997, 10:309-19; Yoda et al., J Med Virol 2000, 60:475-81). MBP is therefore described as a chaperone like molecule (solubilizing agent) (Bach et al., J Mol Biol 2001, 312:79-93; Kapust and Waugh, Protein Sci 1999, 8:1668-74). Presence of MBP may furthermore result in a significantly enhanced yield of a protein of interest if fused to MBP as a fusion partner as compared to the non-fused protein of interest.

However, solubilizing agents (such as glutathion-*S*-transferase, MBP, beta-galactosidase, etc.) in the fusion protein may interfere with the biological function of its fusion partner, i.e. the protein of interest, and may thus lead to a significant decrease in enzymatic activity. This requires cleavage of protein components after protein expression and purification of the mixture in order to obtain a homogeneous gene product. Usually, this step is performed by proteolytic cleavage using a specific protease recognizing a cleavage site located between both fusion protein components. E.g. US 5,643,758 discloses purification of recombinant fusion proteins comprising MBP as solubilizing agent and a protein of interest. Both fusion partners are linked via the protease recognition sequence for blood coagulation factor Xa. Subsequent to purification the protease recognition sequence is cleaved proteolytically, thereby releasing the protein of interest. However, additional purification steps are required after proteolytic cleavage in order to separate the protein of interest from the protease, the solubilizing agent and the unprocessed fusion protein. These steps may significantly decrease purification yields and inherently bear the risk of contamination with proteases and by-products resulting from proteolytic cleavage.

Additionally, fusion proteins comprising solubilizing agents have so far only been proven to work efficiently in prokaryotic host cells, such as *E*. *coli,* even though their potential use in eukaryotic systems has been mentioned before (see e.g. US 5,643,758). However, a wide variety of eukaryotic proteins cannot be expressed at all in functional form in prokaryotic host cells, i.e. in soluble and native conformation, neither alone nor as a component of a fusion protein together with a solubilizing agent, such as MBP.

Summarizing the above, there is still a need in the art for providing an expression vector suitable for both eukaryotic and prokaryotic host cells, which allows to efficiently express a protein of interest at high expression rates, thereby avoiding formation of inclusion bodies and, preferably, additionally avoiding proteolytic cleavage subsequent to purification of the protein of interest.

This problem is solved according to the present invention by an expression vector (A) for enhanced expression of a protein of interest, encoding a fusion protein comprising:
(A1) a "secretory signal sequence";
(A2) a "solubilizing agent";
(A3) a "proteolytic cleavage site";
(A4) a "protein of interest"; and optionally
(A5) a "tag for purification" of the fusion protein.

The expression vector (A) according to the present invention as defined above encodes a fusion protein comprising as component (A1) a "secretory signal sequence". This "secretory signal sequence" provides for secretion of the fusion protein subsequent to expression of same in a host cell. As defined herein a "secretory signal sequence" is a peptide sequence which typically comprises about 10 to 30 amino acids, more preferably about 15 to 30 amino acids. The secretory signal sequence is usually located at the N-terminus of the expressed fusion peptide and enables the fusion peptide to be secreted, i.e. to pass through a cell membrane into the cell culture or extracellular medium or into the periplasmic space (e.g. for bacteria). Examples of secretory signal sequences contained in the fusion protein as encoded by an expression vector (A) according to the present invention may be derived from a secreted protein such as a cytokine, a clotting factor, an immunoglobulin, a secretory enzyme, secreted matrix metalloproteinases (MMP), e.g. a stromelysin leader sequence, from secreted human alkaline phosphatase (SEAP), pro-exendin, e.g. a proexendin-4 leader sequence, or may be derived from pro-helodermin, pro-glucose-dependent insulinotropic polypeptide (GIP), pro-insulin-like growth factor (IGF1), preproglucagon, alpha-1 antitrypsin, insulin-like growth factor 1, human factor IX, human lymphotoxin A (Genbank Accession no. BAA00064), human clusterin (Genbank Accession no. AAP88927). Furthermore, secretory signal sequences may be derived from neuroendocrine protein 7B2 precursor (secretory granule endocrine, protein 1) (secretogranin V) (homo sapiens); accessory gland-specific peptide 95EF precursor (male accessory gland,secretory protein 316) (drosophila melanogaster); antileukoproteinase 1 precursor (ALP) (HUSI-1) (seminal proteinase, inhibitor) (secretory leukocyte protease inhibitor) (BLPI) (mucus, proteinase inhibitor) (MPI) (WAP four-disulfide core domain protein 4), (protease inhibitor WAP4 (homo sapiens); antileukoproteinase 1 precursor (ALP) (secretory leukocyte protease, inhibitor) (mus musculus); complement C1q tumor necrosis factor-related protein 3 precursor (secretory protein CORS26) (homo sapiens); small inducible cytokine A2 precursor (CCL2) (monocyte chemotactic, protein 1) (MCP-1) (monocyte chemoattractant protein-1) (monocyte, chemotactic and activating factor) (MCAF) (monocyte secretory protein, JE) (homo sapiens); CAMPATH-1 antigen precursor (CD52 antigen) (epididymal secretory, protein E5) (canis familiaris); CAMPATH-1 antigen precursor (CD52 antigen) (CDW52) (cambridge, pathology 1 antigen) (epididymal secretory protein E5) (Homo sapiens); chitinase 3-like protein 3 precursor (secretory protein Ym1), (eosinophil chemotactic cytokine) (ECF-L) (mus musculus); chromogranin A precursor (CgA) (pituitary secretory protein I) (SP-I) (bos taurus); pelB signal sequence of the *Erwinia carotovora* pectate lyase having the sequence MKYLLPTAAAGLLLLAAQPAM; ompA signal sequence of the *Escherichia coli* outer membrane protein A having the sequence MKKTAIAIAVALAGFATVAQ; ompF signal sequence of the *Escherichia coli* outer membrane protein F having the sequence MMKRNILAVIVPALLVAGTANA; ompT signal sequence of the *Escherichia coli* protease 7 having the sequence MRAKLLGIVLTTPIAISSFA; phoA signal sequence of the *Escherichia coli* PhoA protein having the sequence MKHSTIALALLPLLFTPVTKA; Ig kappa-chain leader sequence: murine Ig kappa-chain V-J2-C leader sequence having the sequence METDTLLLWVLLLWVPGSTGD; etc. A "secretory signal sequence" as defined herein may further provide for a cleavage site recognized by a signal sequence protease, e.g. a protease as defined below. Such secretory signal sequences are intended to channel the fusion protein though the (inner and outer) cell membrane. They are usually cleaved off from the fusion protein during secretion. A preferred example in the context of the present invention comprises the yeast mating type α-factor secretory signal sequence of *Saccharomyces cerevisiae.* This secretory signal sequence gets removed by the endogenous *Pichia* protease kexin (KEX2) within the yeast secretory pathway.

Furthermore, the expression vector (A) according to the present invention as defined above encodes a fusion protein comprising as component (A2) a "solubilizing agent", selected from a highly soluble and stably expressed protein or a domain thereof (Nygren et al., Trends Biotechnol 1994, 12:184-8; Uhlen et al., Curr Opin Biotechnol 1992, 3:363-9). Proteins or domains thereof that are employed herein as solubilizing agents include e.g. glutathion-S-transferase (GST) (Tudyka and Skerra, Protein Sci 1997, 6:2180-7), thioredoxin (TRX) (LaVallie et al., Biotechnology (NY) 1993, 11:187-93), ubiquitin (Power et al., J Biol Chem 1990, 265:1419-24), protein A (Samuelsson et al., Biochemistry 1994, 33:4207-11), DsbA (Zhang et al., Protein Expr. Purif. 1998, 12:159-65), domain 1 of IF2 protein of *E*. *coli* (Sorensen et al., Protein Expr Purif 2003, 32:252-9), NusA (De Marco et al. (2004), Biochem Biophys Res Commun 322, 766-771) and maltose-binding protein (MBP) (di Guan et al., Gene 1988, 67:21-30; Kapust and Waugh, Protein Sci 1999, 8:1668-74), and chaperones and chaperone cofactors including but not limited to GroES, GroEL, GrpE, DanK, DnaJ, Hsp100, Hsp90, Hsp70, Hsp60, Hsp25 (Hartl and Hayer-Hartl, Science 2002, 295:1852-8), etc.. MBP is a naturally occuring periplasmic protein of *E*. *coli* that is also well expressed in the bacterial cytoplasm. It enhances solubility and stability of proteins fused to it, probably by promoting the correct folding of the fusion partner (Hayhurst, 2000, supra; Pryor, 1997, supra; Jacquet, 1999, supra; Yoda, 2000, supra). Typically, the maltose binding protein (MBP) may be used as a solubilizing agent, i.e. as component (A2), of a fusion protein encoded and expressed by an inventive expression vector (A).

The expression vector (A) according to the present invention as defined above encodes a fusion protein additionally comprising as component (A3) a proteolytic cleavage site. A "proteolytic cleavage site" in the context of the present invention is preferably a peptide sequence which typically comprises about 5 to 30 amino acids and represents a naturally occurring recognition motif including a cleavage site of an endogenously or heterologously expressed protease (A6) as defined below. The "proteolytic cleavage site" in the context of the present invention is intended to allow cleavage of the fusion protein expressed by the inventive expression vector (A) typically subsequent to or, alternatively, during its secretion into the extracellular medium. Cleavage of the fusion protein at its proteolytic cleavage site (A3) upon presence of a corresponding protease (A6) leads to separation of the components linked by the proteolytic cleavage site (A3) and thus to separation of the protein of interest, e.g. from at least its partner components (A1) and (A2). Consequently, the protein of interest (A4) is thereby released into the extracellular medium. Cleavage of the fusion protein at its proteolytic cleavage site typically occurs at the outer membrane of a host cell, i.e. not freely in the extracellular medium.

Proteolytic cleavage sites in the context of the present invention may comprise, without being limited thereto, proteolytic cleavage sites recognized by the proteases Kexin (KEX2) and Furin. Kexin belongs to a family of endoproteases homologous to bacterial subtilisins and mammalian subtilisin like convertases. A human family member is proprotein processing enzyme furin (Rockwell and Thorner, Trends Biochem Sci 2004; 29:80-7). (Furin is also known as PACE, see U.S. Pat. No. 5,460,950). Furin is a ubiquitously expressed protease that resides in the trans-golgi and processes protein precursors before or during their secretion. Furin cleaves at the COOH-terminus of its consensus recognition sequence, Arg-X-X-Arg (SEQ ID NO: 1), such as Arg-X-Lys/Arg-Arg (SEQ ID NO: 2) or Arg-Gln/Tyr-Lys/Arg-Arg (SEQ ID NO: 3). Proteolytic cleavage sites within the scope of this invention fitting the Arg-X-Lys/Arg-Arg motif (SEQ ID NO: 1) and capable of being cleaved by furin may be derived e.g. from precursors sequences of a wide variety of proteins. These include e.g. growth factors, their receptors, plasma proteins involved in blood-clotting and complement system, matrix metalloproteinases, viral-envelope glycoproteins and bacterial exotoxins (Rockwell and Thorner, 2004, supra). An optimal *in vitro* furin recognition site (fur) has been identified by incubating recombinant furin with peptidyl methyl coumarin amide substrates comprising of an acetylated hexapeptide recognition motif followed by a fluorogenic group (Krysan et al., J Biol Chem 1999; 274:23229-34). This optimal furin recognition motif is also a substrate of the above mentioned *Pichia* homolog kexin (KEX2). More preferably, furin (and kexin) cleavage sites comprise the sequences Arg-Ala-Arg-Tyr-Lys-Arg (SEQ ID NO: 4) (herein also termed "fur") or Arg-Ala-Arg-Tyr-Lys-Arg-Ser (SEQ ID NO: 5) (herein also termed furS). Proteolytic cleavage of an optimized furin cleavage site according to SEQ ID NO: 5 with Furin or Kexin (KEX2) may lead to a serine moiety remaining at the N-terminal end of the cleaved downstream peptide sequence, e.g. the N-terminal end of the protein of interest (A4). Such serine may, in some cases, influence or even inhibit the catalytic activity of the downstream protein sequence, e.g. the protein of interest (A4). Thus, alternative proteolytic cleavage sites for furin or kexin protease may preferably be used in these cases, lacking a serine at this position (e.g. SEQ ID NO: 4).

Proteolytic cleavage sites in the context of the present invention may also comprise, without being limited thereto, proteolytic cleavage sites of subtilisins (including PC2, PC1/PC3, PACE4, PC4, PC5/PC6, LPC/PC7IPC8/SPC7 and SKI-1; Nakayama, Biochem. J., 327:625-635 (1997)), the proteolytic cleavage site of chymotrypsin, or proteolytic cleavage sites of enterokinases (see U.S. Pat. No. 5,270,181), e.g. enterokinase recognition motifs Asp-Asp-Asp-Aps-Lys* (Lu et al. (1999), J Mol Biol 292, 361-373) and Asp/Glu-Arg-*Met (Boulware and Daugherty, 2006, Proc Natl Acad Sci U S A 103, 7583-7588), the proteolytic cleavage site of coagulation factor Xa, e.g. the recognition motif Glu-Gly-Arg* (Cho et al. (1984), Biochemistry 23, 644-650; Matthews and Wells (1993), Science 260, 1113-1117.), the proteolytic cleavage site of Thrombin, e.g. the recognition motif Leu-Val-Pro-Arg*Gly-Ser (Su et al. (2004), Protein Eng Des Sel 17, 647-657.), the proteolytic cleavage site of tobacco etch virus (TEV) protease, e.g. the recognition motif Glu-Asn-Leu-Tyr-Phe-Gln*Gly/Ser (Nallamsetty et al. (2004), Protein Expr Purif 38, 108-115), the proteolytic cleavage site of tobacco vein mottling virus (TVMV) protease, e.g. the recognition motif Glu-Thr-Val-Arg-Phe-Gln*Gly/Ser (Nallamsetty *et al.* (2004), supra), the proteolytic cleavage site of Granzyme B, e.g. the recognition motif Ile-Glu-Pro-Asp* (Harris et al. (1998), J Biol Chem 273, 27364-27373), the proteolytic cleavage site of Caspase-3, e.g. the recognition motif: Asp-Glu-Val-Asp* (Talanian et al. (1997), J Biol Chem 272, 9677-9682), etc..

Proteolytic cleavage sites (A3) in the context of the present invention may furthermore comprise a sequence showing about 80%, more preferably about 90% and most preferably about 95 or even about 99 % sequence identity with a proteolytic cleavage site as defined above, more preferably with the unmodified, i.e. naturally occurring, proteolytic cleavage site of a protease as defined below.

The term "sequence identity" as defined herein means that the sequences are compared as follows. To determine the percent identity of two amino acid sequences, the sequences can be aligned for optimal comparison purposes (e.g. gaps can be introduced in the sequence of a first amino acid sequence). The amino acids at corresponding amino acid positions can then be compared. When a position in the first sequence is occupied by the same amino acid as the corresponding position in the second sequence, then the molecules are identical at that position. The percent identity between the two sequences is a function of the number of identical positions shared by the sequences. E.g. where a particular peptide is said to have a specific percent identity to a reference polypeptide of a defined length, the percent identity is relative to the reference peptide. Thus, a peptide that is 50% identical to a reference polypeptide that is 100 amino acids long can be a 50 amino acid polypeptide that is completely identical to a 50 amino acid long portion of the reference polypeptide. It might also be a 100 amino acid long polypeptide, which is 50% identical to the reference polypeptide over its entire length. Of course, other polypeptides will meet the same criteria. Such a determination of percent identity of two sequences can be accomplished using a mathematical algorithm. A preferred, non-limiting example of a mathematical algorithm utilized for the comparison of two sequences is the algorithm of Karlin *et al.* (1993), PNAS USA, 90:5873-5877. Such an algorithm is incorporated into the NBLAST program, which can be used to identify sequences having the desired identity to an amino acid sequence as defined herein. To obtain gapped alignments for comparison purposes, Gapped BLAST can be utilized as described in Altschul et al. (1997), Nucleic Acids Res., 25:3389-3402. When utilizing BLAST and Gapped BLAST programs, the default parameters of the respective programs (e. g., NBLAST) can be used. The sequences further may be aligned using Version 9 of the Genetic Computing Group's GAP (global alignment program), using the default (BLOSUM62) matrix (values-4 to +11) with a gap open penalty of -12 (for the first null of a gap) and a gap extension penalty of -4 (per each additional consecutive null in the gap). After alignment, percentage identity is calculated by expressing the number of matches as a percentage of the number of amino acids in the claimed sequence. The described methods of determination of the percent identity of two amino acid sequences can be applied correspondingly to nucleic acid sequences.

The proteolytic cleavage site (A3) as defined above is typically positioned between the solubilizing agent (A2) and the protein of interest (A4) of a fusion peptide as expressed by the expression vector (A) according to the present invention. The proteolytic cleavage site (A3) may be positioned either between the C-terminus of the protein of interest (A4) and the N-terminus of the solubilizing agent (A2), or, more preferably, between the C-terminus of the solubilizing agent (A2) and the N-terminus of the protein of interest (A4). The proteolytic cleavage site (A3) may be linked to the solubilizing agent (A2) and/or to the protein of interest (A4) directly or via a linker. A "linker" in the present context is preferably an oligo- or polypeptide and may be used to link any of the components (A1), (A2), (A3) or (A4). Preferably, a linker has a length of 1-10 amino acids, more preferably a length of 1 to 5 amino acids and most preferably a length of 1 to 3 amino acids. Advantageously, the linker does not have any secondary structure forming properties, i.e. has no α-helix or β-sheet structure forming tendency, e.g. if the linker is composed of at least 35 % of glycin residues. A linker may be typically be an all-glycine sequence, e.g. GG, GGG, GGGG, GGGGG, etc..

Furthermore, the expression vector (A) according to the present invention as defined above (A) encodes a fusion protein comprising as component (A4) a protein of interest. A "protein of interest" (A4) in the context of the present invention may be selected from any recombinant or naturally occurring protein known in the art. Without being limited thereto the protein of interest (A4) may be selected e.g. from growth hormones or growth factors, for example to promote growth in a transgenic animal, such as TGFα and insuline like growth factors (IGFs), etc.. Similarly, the protein of interest (A4) may be selected from molecules that effect metabolism and hematopoiesis such as α-anti-trypsin, insulin, LDL-receptor, erythropoietin (EPO), GATA-1, etc., or molecules such as factors VIII and XI of the blood clotting system, etc.. Similarly, the protein of interest (A4) may be selected from enzymes such as [beta]-galactosidase (lacZ), DNA restriction enzymes (e.g. EcoRI, HindIII, etc.), DNA polymerases (e.g. Taq, Pfu, Vent, etc.) lysozyme, etc., or proteases and protease inhibitors such as papain, bromelain, keratinases, trypsin, chymotrypsin, pepsin, rennin (chymosin), suizym, nortase, etc.. Similarly, the protein of interest (A4) may be selected from proteins that stimulate or inhibit cell signalling, e.g. cytokines, antibodies. E.g. the protein of interest (A4) may be selected from e.g. cytokines from class I cytokine family (hematopoietin receptor family) having 4 positionally conserved Cysteine residues (CCCC) and a conserved sequence motif Trp-Ser-X-Trp-Ser (WSXWS), wherein X is a non-conserved amino acid. The class I cytokine receptor family includes the GM-CSF subfamily, e.g. IL-3, IL-5, GM-CSF, the IL-6 subfamily, e.g. IL-6, IL-11, IL-12, or the IL-2 subfamily, e.g. IL-2, IL-4, IL-7, IL-9, IL-15, etc., or cytokines IL-1α, IL-1β, IL-10 etc.. Similarly, the protein of interest (A4) may be selected from cytokines from class II cytokine receptor family (interferon receptor family) also having 4 positionally conserved Cysteine residues (CCCC) but no conserved WSXWS motif. The class II cytokine receptor family includes IFN-α, IFN-β, IFN-γ, etc.. The protein of interest (A4) may also be selected from cytokines from the tumor necrosis family, e.g. TNF-α, TNF-β, TNF-RI, TNF-RII, CD40, Fas, etc., or from cytokines from the chemokine family, having 7 transmembrane helices and interacting with G-protein, e.g. IL-8, MIP-1, RANTES, CCR5, CXR4, etc.. The protein of interest (A4) may additionally be selected from antibodies, preferably from monoclonal antibodies, single-chain antibodies (sc-antibodies), such as scFv, e.g. anti-ErbB2 scFv(FRP5), pathogen-specific antibodies, tumor-specific antibodies, etc.. The protein of interest (A4) may further be an apoptotic factor or an apoptosis related enzyme including AIF, Apaf e.g. Apaf-1, Apaf-2, Apaf-3, or APO-2 (L), APO-3 (L), Apopain, Bad, Bak, Bax, Bcl-2, Bcl-x_{L}, Bcl-x_{S}, bik, CAD, Calpain, Caspases e.g. Caspase-1, Caspase-2, Caspase-3, Caspase-4, Caspase-5, Caspase-6, Caspase-7, Caspase-8, Caspase-9, Caspase-10, Caspase-11, ced-3, ced-9, c-Jun, c-Myc, crm A, cytochrome C, CdR1, DcR1, DD, DED, DISC, DNA-PK_{CS}, DR3, DR4, DR5, FADD/MORT-1, FAK, Fas (Fas-ligand CD95/fas (receptor)), FLICE/MACH, FLIP, Fodrin, fos, G-Actin, Gas-2, Gelsolin, granzyme A/B, ICAD, ICE, JNK, Lamin A/B, MAP, MCL-1, Mdm-2, MEKK-1, MORT-1, NEDD, NF-_{κ}B, NuMa, p53, PAK-2, PARP, Perforin, PITSLRE, PKCδ, pRb, Presenilin, prICE, RAIDD, Ras, RIP, Sphingomyelinase, thymidine kinase from Herpes simplex, TRADD, TRAF2, TRAIL-R1, TRAIL-R2, TRAIL-R3, Transglutaminase, etc.. The protein of interest (A4) may further be a bacterial or plant toxin (e.g. ribosome inactivating protein, etc.), an immunotoxin, a vaccine or antigen (e.g. pathogen-derived antigens, tumor-associated antigens), etc.. A protein of interest (A4) as defined above also includes any fragment of the above proteins, i.e. a fragment thereof showing a sequence identity of at least 40%, 50%, 60%, 70%, 80%, 90%, 95% or 99% with their corresponding non-modified protein of interest (A4) as defined above. Additionally, these fragments preferably retain the biological or enzymatic function of their corresponding non-modified protein of interest (A4) as defined above.

Additionally, the expression vector (A) according to the present invention as defined above (A) encodes a fusion protein comprising as optional component (A5) at least one "tag for purification" of the protein of interest (A4) subsequent to secretion and proteolytic cleavage of the fusion protein. A "tag for purification" (A5) in the context of the present invention may be of any variety of tags suitable for purification of recombinant proteins such as a hexahistidine-tag (his-tag, polyhistidine-tag), a streptavidine tag (strep-tag), a SBP-tag (a streptavidine binding tag), a GST(glutathione-S-transferase)-tag, etc., or by means of an antibody epitope (an antibody binding tag), e.g. a myc-tag, a Swa11-epitope, a FLAG-tag, etc.. Tags as mentioned above are preferably located at the C-terminus of the protein of interest (A4) as defined above and shall typically not be cleaved off by protease cleavage acting on the proteolytic cleavage site (A3) as defined above. Furthermore, tags as mentioned above are preferably selected such as not to interfere with the biological functionality, e.g. or enzymatical activity or a binding activity, of a protein of interest (A4) as defined above. Thus, non-bulky tags are preferably selected, such as a hexahistidine-tag, a streptavidine tag (strep-tag), a SBP-tag (a streptavidine binding tag), a or an antibody epitope, e.g. a myc-tag, a Swa11-epitope, a FLAG-tag, etc..

Generally, components (A1), (A2), (A3), (A4) and optionally (A5) of the fusion protein as encoded by the inventive expression vector (A) as defined herein may be arranged as suitable, i.e. in any order allowing to express and secrete a fusion protein and to carry out proteolytic cleavage in order to have its components ((A2), (solubilizing agent) and (A4), protein of interest)) separated at the end of the secretion process. Typically, its component (A1) will be located that the very N-terminus of the fusion protein encoded. According to a preferred embodiment, the expression vector (A) according to the present invention as defined above encodes a fusion protein as defined above comprising from N- to C-terminus (A1) a secretory signal sequence as defined above; (A2) a solubilizing agent as defined above; (A3) a proteolytic cleavage site as defined above; (A4) a protein of interest as defined above; and optionally (A5) a tag for purification of the protein of interest as defined above. However, component (A4) may also be exchanged by component A2 and *vice versa.*

In order to release the protein of interest (A4) of the fusion protein encoded by the inventive expression vector (A) as defined above proteolytic cleavage at the proteolytic cleavage site (A3) is required subsequent to or during secretion of the fusion protein. According to the present invention, proteolytic cleavage at proteolytic cleavage site (A3) is carried out using an endogenously or heterogeneously expressed protease as defined below. This protease may be either be encoded by an expression vector (A) as defined above, or, independent upon expression vector (A), by an additional expression vector B. Alternatively, the protease may be expressed endogenously by the host cells used herein, i.e. the host cells used for transfection of the inventive expression vector (A) as defined above. According to another alternative (which may be applied, if required, additionally to the above alternatives), the protease may be added extracellularly to the host cell culture medium, if expression of the fusion protein shall be carried out *in vitro.*

Thus, according to one embodiment, the expression vector (A) according to the present invention as defined above may optionally encode a protease (A6), capable of cleaving the proteolytic cleavage site (A3) subsequent to or during secretion of the fusion protein. The protease (A6) may be selected from any protease suitable for cleaving the proteolytic cleavage site (A3) as defined above. Without being limited thereto, such a protease (A6) preferably comprises proteases naturally occurring in eukaryotic or prokaryotic host cells, more preferably, the naturally occurring proteases of the corresponding host cells used in the present invention, and most preferably membrane standing proteases. Such proteases include e.g. Kexin (KEX2), a protease of a family of endoproteases with homology to bacterial subtilisins and subtilisin-like proteins. Endoproteases of this family, which are also encompassed herein, process their substrates by proteolysis at paired basic amino acids (Fuller et al., Science 1989, 246:482-6). Such proteases furthermore include e.g. Furin, the so far best characterized enzyme of the mammalian subtilisin-like family of convertases. Enzymatic activity and substrate specificity of furin have been evaluated by cellular co-expression and *in vitro* studies. Other preferred proteases include the Coagulation factor Xa (Cho *et al*., 1984, supra; Matthews and Wells, 1993, supra), Thrombin (Su *et al*., 1004, supra), enterokinase(s) (Lu *et al*., 1999, supra, Boulware and Daugherty, 2006, supra), tobacco etch virus (TEV) protease (Nallamsetty *et al*., 2004 , supra), tobacco vein mottling virus (TVMV) protease, Granzyme B (Harris *et al*., 1998, supra), Caspase-3 (Talanian *et al*., 1997, supra), etc.. Any of the above proteases (A6) may be expressed together with an ER retention signal (KDEL/HDEL) or may occur as membrane bound protease, e.g. by carrying a transmembrane domain. Furthermore, a protease (A6) as defined above may additionally or alternatively contain a secretory signal sequence as defined above, if the protease is a heterologously expressed protease, so that it is normally secreted along the secretory pathway of the host cell as used herein. This allows for proteolytic cleavage of the proteolytic cleavage site (A3) of the fusion protein as defined above directly at the membrane of the host cell subsequent or during secretion of the fusion protein and thus enables release of the protein of interest (A4). If proteases as defined above, do not naturally contain an ER retention signal, a transmembrane domain and/or a secretory signal sequence, these proteases may be modified accordingly using methods generally known in the art to a skilled person (see e.g. Maniatis *et al.* (2001), supra).

According to another embodiment, the present invention provides a second expression vector (B) encoding a protease as defined above for protease (A6), capable of cleaving the proteolytic cleavage site (A3) subsequent to or during secretion of the inventive fusion protein. According to this embodiment, the expression vector (A) as defined above does not contain a protease (A6) as an additional component.

According to a further embodiment of the present invention, a protease (A6) as defined above, may be endogenously expressed by a host cell used herein. i.e. the host cell as used herein already encodes and expresses the protease (A6) without the necessity of transfecting an expression vector encoding this protease. Thus, according to this specific embodiment, the expression vector (A), typically does not contain the protease (A6) as an additional component. However, according to an alternative of this embodiment, an expression vector (B) as defined above may be additionally transfected into the host cell, or expression vector (A) may contain protease (A6) as an additional component, if enhanced expression of protease (A6) is desired in comparison to the typical expression of protease (A6) as provided by the host cell.

Finally, according to another embodiment, the protease as defined above for protease (A6) may be added extracellularly to the host cell culture medium, if expression of the fusion protein is carried out *in vitro*. According to this specific embodiment, additionally any of the above alternatives may be employed, i.e. the protease may be encoded by an inventive expression vector (A) as component (A6), or may be encoded separately by an inventive expression vector (B), or may be expressed endogenously by a host cell as used herein transfected with an inventive expression vector (A).

An inventive expression vector (A) encoding a fusion protein as defined above, and an expression vector (B) as defined above, may comprise additional elements suitable for directing expression of the fusion protein encoded by expression vector (A) and (optionally) of the protease as defined above for expression vectors (A) or (B). E.g. the inventive expression vectors (A) and (B) may contain specific regulatory elements, which e.g. control expression of the encoded amino acid sequences. Such regulatory elements are e.g. 1) specific to a tissue or region of the body; 2) constitutive; 3) glucose responsive; and/or 4) inducible/regulatable. Regulatory elements herein are preferably selected from regulation sequences and origins of replication (if the vectors are replicated autonomously). Regulation sequences as applicable herein are any elements known to a skilled person having an impact on expression on transcription and/or translation of nucleic acid sequences encoding a fusion protein as defined above and, generally, a protease as defined above. These regulation sequences include, apart from promoter sequences, so-called enhancer sequences, which may lead to an increased expression due to enhanced interaction between RNA polymerase and DNA. Further regulation sequences of expression vectors (A) and (B) as defined above include transcriptional regulatory and translational initiation signals, so-called "terminator sequences", etc. or partial sequences thereof.

Generally, any naturally occurring promoter may be contained in inventive expression vectors (A) and (B) as defined above. Such promoters may be selected from any eukaryotic or prokaryotic promoters, e.g. viral, plant, bacterial, fungal, human or animal promoters. Suitable promoters include, for example, the cytomegalovirus promoter, the lacZ promoter, the gal 10 promoter and the AcMNPV polyhedral promoter, promoters such as cos-, tac-, trp-, tet-, trp-tet-, Ipp-, lac-, Ipp-lac-, laclq-, T7-, T5-, T3-, gal-, trc-, ara-, SV40-, SP6, I-PR-or the I-PL-promoter, advantageously being found in gram-negative bacteria. Additionally, promoters may be obtained from gram-positive promoters such as amy and SPO2, yeast promoters, such as the methanol inducible alcohol oxidase I (AOX1) promoter, or ADC1, MFa, AC, P-60, CYC1, GAPDH promoters, or mammalian promoters such as the cytomegalovirus (CMV) promoter, muscle-specific promoters including mammalian muscle creatine kinase (MCK) promoter, mammalian desmin promoter, mammalian troponin I (TNNI2) promoter, or mammalian skeletal alpha-actin (ASKA) promoter, or liver type pyruvate kinase promoters, particularly those fragments which run (-183 to +12) or (-96 to +12) (Thompson, et al. J Biol Chem, (1991). 266:8679-82.; Cuif, et al., Mol Cell Biol, (1992). 12:4852-61); the spot 14 promoter (S14, -290 to +18) (Jump, et al., J. Biol Chem, (1990). 265:3474-8); acetyl-CoA carboxylase (O'Callaghan, et al., J. Biol Chem, (2001). 276:16033-9); fatty acid synthase (-600 to +65) (Rufo, et al., J Biol Chem, (2001). 28:28); and glucose-6-phosphatase (rat and human) (Schmoll, et al., FEBS Left, (1996). 383:63-6; Argaud, et al., Diabetes, (1996). 45:1563-71), or promoters from CaM-Kinasell, Nestin, L7, BDNF, NF, MBP, NSE, beta-globin, GFAP, GAP43, tyrosine hydroxylase, Kainat-receptor-subunit 1, glutamate-receptor-subunit B, or human ubiquitin promoter B (ubiB human), human ferritin H promoter (FerH), etc.. Particularly suitable promoters are of human or mammalian origin. Finally, synthetic promoters may be used advantageously. Promoter sequences, as contained in inventive expression vectors (A) and (B) as defined above may also be inducible for *in vitro* purposes, to allow modulation of expression (e.g. by the presence or absence of nutrients or other inducers in the growth medium). One example is the lac operon obtained from bacteriophage lambda plac5, which can be induced by IPTG. Typically, a promoter as defined above may be positioned upstream of the at least one encoding nucleic acid sequence, i.e. a nucleic acid sequence encoding a fusion protein as defined above and/or a protease as defined above.

Enhancer sequences for upregulating expression of a fusion protein as defined above and/or a protease as defined above in inventive expression vectors (A) and (B) as defined above are preferably another constituent of these expression vectors. Such enhancer sequences are typically located in the non-coding 3' region of the nucleic acid sequences encoding the fusion protein and/or the protease. Enhancer sequences as employed in inventive expression vectors (A) and (B) as defined above may be obtained from any eukaryotic or prokaryotic host, e.g. bacterial, fungal, plant, human or animal (e.g. mammalian) hosts, preferably in association with the corresponding promoters as defined above. Enhancer elements which will be most useful in the present invention are those which are glucose responsive, insulin responsive and/or liver specific. Enhancer elements may include the CMV enhancer (e.g. linked to the ubiquitin promoter (Cubi)); one or more glucose responsive elements, including the glucose responsive element (G1 RE) of the liver pyruvate kinase (L-PK) promoter (-172 to -142); and modified versions with enhanced responsiveness (Cuif *et al.,* supra; Lou, et al., J. Biol Chem, (1999). 274:28385-94); G1 RE of L-PK with auxiliary L3 box (-172 to -126) (Diaz Guerra, et al., Mol Cell Biol, (1993). 13:7725-33; modified versions of G1RE with enhanced responsiveness with the auxiliary L3 box; carbohydrate responsive element (ChoRE) of S 14 (-1448 to -1422), and modifications activated at lower glucose concentrations (Shih and Towle, J Biol Chem, (1994). 269:9380-7; Shih, et al., J Biol Chem, (1995). 270:21991-7; and Kaytor, et al., J Biol Chem, (1997). 272:7525-31; ChoRE with adjacent accessory factor site of S 14 (-1467 to -1422) [*et al.,* supra]; aldolase (+1916 to +2329)(Gregori et al., J Biol Chem, (1998). 273:25237-43; Sabourin, et al., J. Biol Chem, (1996). 271:3469-73; and fatty acid synthase (-7382 to -6970) (Rufo*, et al.,* supra.), more preferably insulin responsive elements such as glucose-6-phosphatase insulin responsive element (-780 to -722) [Ayala et al., Diabetes, (1999). 48:1885-9; and liver specific enhancer elements, such as prothrombin (940 to -860) [Chow et al., J Biol Chem, (1991) 266: 18927-33; and alpha-1-microglobulin (-2945 to -2539) [Rouet et al., Biochem J, (1998). 334:577-84), Muscle-specific enhancers such as mammalian MCK enhancer, mammalian DES enhancer, and vertebrate troponin I IRE (TNI IRE, herein after referred to as FIRE) enhancer. Finally, a SV40 enhancer sequence may also be included. These enhancer elements may be used along with promoters as defined above. E.g. such promoter/enhancer combinations include, without being limited thereto, e.g. the cytomegalovirus (CMV) promoter and the CMV enhancer, the CMV enhancer linked to the ubiquitin promoter (Cubi), the group of liver-specific enhancer elements comprising human serum albumin [HSA] enhancers, human prothrombin [HPrT] enhancers, alpha-1 microglobulin [A1MB] enhancers, and intronic aldolase enhancers used in combination with their corresponding promoters, or HSA enhancers used in combination with a promoter selected from the group of a CMV promoter or an HSA promoter, enhancer elements selected from the group consisting of human prothrombin [HPrT] and alpha-1 microglobulin [A1MB] used in combination with the CMV promoter enhancer elements selected from the group consisting of human prothrombin [HPrT] and alpha-1 microglobulin [A1MB] used in combination with the alpha-1-anti trypsin promoter, etc..

Furthermore, the inventive expression vectors (A) and (B) as defined above may contain transcriptional and/or translational signals, preferably recognized by an appropriate host, such as transcriptional regulatory and translational initiation signals. Transcriptional and/or translational signals may be obtained from any eukaryotic, prokaryotic, viral, bacterial, fungal or plant origin, preferably human or animal hosts, e.g. mammalian hosts, preferably in association with the corresponding promoters as defined above. A wide variety of transcriptional and translational regulatory sequences may be employed therefore, depending upon the nature of the host cell. To the extent that the host cell recognizes the transcriptional regulatory and translational initiation signals contained in inventive expression vector (A), encoding a fusion protein as defined above, and, optionally, in inventive expression vector (B), encoding a protease as defined above, the naturally occurring 5' region(s) adjacent to components of the fusion protein and/or to the protease may be retained and employed for transcriptional and translational regulation in inventive expression vectors (A) and (B) as defined above. This region typically will include those sequences involved with initiation of transcription and translation, such as the TATA box, capping sequence, CAAT sequence, and the like. Typically, this region will be at least about 150 base pairs long, more typically about 200 bp, and rarely exceeding about 1 to 2 kb. Furthermore, transcriptional initiation regulatory signals may be selected that allow to control repression or activation such that expression of the nucleic acid sequences of an inventive expression vector (A) as defined above encoding a fusion protein and, optionally, of an inventive expression vector (B) as defined above encoding a protease can be modulated. One such controllable modulation technique is the use of regulatory signals that are temperature-sensitive in order to repress or initiate expression by changing the temperature. Another controllable modulation technique is the use of regulatory signals that are sensitive to certain chemicals (e.g. IPTG). Transcription and/or translational signals also include transcriptional termination regulatory sequences, such as a stop signal and a polyadenylated region. Preferably, transcriptional termination regulatory sequences are located in the non-coding 3' region of the at least one nucleic acid sequence as defined above. Suitable termination sequences include, for example, the bovine growth hormone, SV40, lacZ, EF1 alpha and AcMNPV polyhedral polyadenylation signals, etc..

In addition to regulation sequences as defined above the inventive expression vectors (A) and (B) typically comprise an origin of replication. Suitable origins of replication include, without being limited thereto, e.g. ColE1, pSC101, SV40, pMPI (ori pMPI) and M13 origins of replication, etc..

Finally, the inventive expression vectors (A) and (B) as defined above may contain (selection) marker genes for selective expression in *in vitro* cell cultures. Such (selection) marker genes preferably allow phenotypic selection of transformed host cells. A (selection) marker gene may provide prototrophy to an auxotrophic host, biocide resistance (e.g. antibiotic resistance genes) and the like. The selectable marker gene may either be contained in inventive expression vectors (A) and (B) as defined above or may be introduced into the same cell by co-transfection. Examples of selectable markers confer resistance to the antibiotics ampicillin, hygromycin, kanamycin, neomycin, and the like or any selection marker which is not based on antibiotic selection.

The inventive expression vectors (A) and (B) as defined above may occur as linear or circular DNA or RNA, e.g. mRNA or cDNA, as naked RNA or DNA and/or as part of a suitable nucleic acid sequence. In the context of the present invention a suitable nucleic acid sequence includes e.g. a DNA element, that provides autonomously replicating extrachromosomal plasmids derived from animal viruses (e.g. bovine papilloma virus, polyomavirus, adenovirus, or SV40, etc.). A second class of nucleic acid sequences as used herein relies upon integration of these nucleic acid sequences into the host cell chromosome. Such suitable nucleic acids are known to a skilled person and may be reviewed e.g. in "Cloning Vectors" (Eds. Pouwels P. H. et al. Elsevier, Amsterdam-New York-Oxford, 1985, ISBN 0 444 904018). Suitable nucleic acids are also intended to include any suitable nucleic acid sequence known to a skilled person, such as plasmids, phages, viruses such as SV40, CMV, Baculo virus, Adeno virus, Sindbis virus, transposons, IS-elements, phasmids, phagemides, cosmides, linear or circular DNA or RNA. Linear DNA is typically used for integration in mammalian cells. Preferably, the type of nucleic acid sequences used in the context of the present invention corresponds to the specific host cell requirements. Suitable commercially available nucleic acids include pSPORT, pBluescriptllSK, pBIIKS, pPIC9, pGEX, pMAL, pFLAG, pCR2.1, the baculovirus expression vector pBlueBac, and the prokaryotic expression vector pcDNAII, all of which may be obtained from Invitrogen Corp., San Diego, CA.

The inventive expression vectors (A) and (B) as defined above may be contained in a suitable host cell or, if appropriate, transfected into a suitable host cell. Suitable host cells are to be understood herein as any cell, allowing expression of the fusion protein and, generally, the protease as encoded by inventive expression vectors (A) and, optionally, (B) as defined above. Any cultivated prokaryotic or eukaryotic host cell, e.g. bacterial, fungal, plant, human and animal host cells, may be used as a host cell. Preferred prokaryotic host cells may be e.g. derived from bacteria, such as *Escherichia coli,* including e.g. XL1-blue, or *B. subtilis,* salmonella, pneumococcus, etc., or from algae, fungae, etc.. Alternatively, eukaryotic host cells, e.g. cells from multi-cellular organisms, may be selected. Compared to prokaryotic expression systems, the advantages of eukaryotic systems are adequate folding, processing and posttranslational modification of heterologously expressed eukaryotic proteins. Cells from eukaryotic organisms are particularly preferred, if posttranslational modifications, e.g. glycosylation of the encoded proteins, are required (N and/or O coupled). In contrast to prokaryotic cells, higher eukaryotic cells may permit these modifications to occur. The skilled person is aware of a plurality of established host cells suitable for this purpose. Typical examples of suitable eukaryotic host cells include yeasts such as Saccharomyces *cerevisiae* (Stinchcomb *et al*., Nature, 282:39, (1997)), Schizosaccharomyces *pombe,* Pichia *pastoris*, including e.g. GS115, etc.. Yeast host cells systems offer an attractive alternative to prokaryotic host cells, because they combine many advantages of other eukaryotic host cells, like folding, processing and posttranslational modification of heterologous proteins, with the ability to produce high protein yields ranging up to grams per liter culture volume. As an example, the methylotrophic yeast *Pichia pastoris* as a eukaryote has been successfully used to express a variety of different proteins in native and functional form (Cereghino and Cregg, FEMS Microbiol Rev 2000, 24:45-66; Cregg et al., Mol Biotechnol 2000, 16:23-52). *Pichia pastoris* has many advantages of eukaryotic expression systems, like folding, processing and posttranslational modification of heterologous proteins. Compared to higher eukaryotes, protein yields obtained in *Pichia pastoris* expression cultures are relatively high, ranging up to grams per liter culture volume. In *Pichia pastoris,* heterologously expressed proteins can be secreted into the culture medium by fusion to the yeast mating type α-factor signal peptide. During export of the protein, the signal sequence is removed by the *Pichia* protease kexin (*KEX2* gene product, Kex2p). During secretion, glycosylation sites become glycosylated; in contrast to proteins expressed in *Saccharomyces cerevisiae* however, no hyperglycosylation is observed and the glycosylation pattern is found to resemble that of human proteins (Romanos et al., Yeast, 8(6): 423-88 (1992)). Other examples of suitable eukaryotic cells include mammalian host cells, e.g. PMBCs, lymphocytes or 293T (embryonic kidney cell line), HeLa (human cervix carcinoma cells) and further host cells, in particular host cells established for laboratory use, such as HEK293-, Sf9-, CHO(Chinese hamster ovary)- or COS-cells. Furthermore, the inventive expression vector (A) may be contained in or transfected into cells taken from a patient/animal to be treated (autologous cells). Autologous cells may be isolated from any tissue or fluid derived from a patient to be treated, e.g. from any organ, blood, lymph, nerve tissue, stem cells, bone marrow cells, etc., according to standard methods known to a skilled person and typically well known in the art. Use of autologous cells for e.g. therapeutic purpose allows to express the fusion protein and, generally, the protease encoded by expression vectors (A) and, optionally, (B), as defined above without evoking any undesired immune response by the patient/animal to be treated.

Transfection of the expression vectors (A) and, optionally, (B) into a suitable host cell as defined above may be carried out by any transfection method known in the art (see e.g. Maniatis *et al.,* 2001, supra). Such transfection methods include spontaneous uptake methods, conventional chemical methods, such as the polyamidoamine dendrimer method, the DEAE-dextran method or the calcium phosphate method, physical methods such as microinjection, electroporation, ballistic particle delivery, etc..

As another embodiment, the present invention provides an *in vitro* or *in vivo* method for enhanced expression of a protein of interest comprising following steps:
(1) optionally generating an inventive expression vector (A), encoding a fusion protein as defined above, and, optionally, an inventive expression vector (B), encoding a protease, as defined above;
(2) optionally transfecting a suitable host cell as defined above with the inventive expression vector (A) and, optionally, with the inventive expression vector (B), both generated in step 1;
(3) expressing the fusion protein and, generally, the protease;
(4) secreting the fusion protein and, generally, the protease; and
(5) proteolytically cleaving the fusion protein at its proteolytic cleavage site using a protease according to steps (3) and (4) or as defined herein.

The inventive *in vitro* or *in vivo* method for enhanced expression of a protein of interest comprises an optional step (1) of preparing an inventive expression vector (A) and optionally the inventive expression vector (B) as defined herein. Methods for preparation of nucleic acids are well known in the art and may be applied for preparation of inventive expression vectors (A) and/or (B) by a skilled person as suitable (see e.g. Maniatis *et al.* (2001) supra). If the inventive method is applied *in vitro,* i.e. for enhanced expression of a protein of interest *in vitro,* according to a first alternative an inventive expression vector (A) is preferably prepared, encoding the fusion protein and the protease within the inventive expression vector. According to a second alternative, the fusion protein and the protease may be encoded by two different expression vectors (A) and (B), i.e. the fusion protein may be encoded by inventive expression vector (A) as defined above, wherein expression vector (A) does not encode the protease, and the protease may be encoded by inventive expression vector (B). According to a third alternative, the inventive expression vector (A) may just encode the fusion protein as defined above but typically no protease (A6) as defined above and typically no expression vector (B) is provided. According to this third alternative a protease as defined above, may be endogenously expressed by a host cell used herein. I.e. the host cell as used herein already encodes and expresses the protease without the necessity of transfecting an expression vector (A) or (B) encoding this protease. However, according to this alternative, an expression vector (B) as defined above may be additionally transfected into the host cell, or expression vector (A) may contain protease (A6) as an additional component, if enhanced expression of protease (A6) is desired in comparison to the typical expression of protease (A6) as provided by the host cell. The protease also may be added exogenously to the cell culture containing the inventive expression vector (A), i.e. into the extracellular culture supernatant, if no or not sufficient protease is (endogeneously) expressed by the host cell. If the inventive method is to be applied *in vivo*, i.e. for enhanced expression of a protein of interest *in vivo*, the first and second alternative as defined above may typically be used, i.e. the fusion protein and the protease may be encoded by the same expression vector (A) or by inventive expression vectors (A) and (B). However, the third alternative may also be applied *in vivo,* provided that the (administered) host cell endogenously expresses the protease, since no protease can be added *in vivo* extracellularly

According to optional step (2) of the inventive *in vitro* or *in vivo* method for enhanced expression of a protein of interest a suitable host cell as defined herein may be transfected (*in vitro* or *in vivo*) with the inventive expression vector (A) and, optionally, with the inventive expression vector (B), both generated in step 1. *In vitro* transfection of suitable host cells may be accomplished by any method known to a skilled person (see e.g. Maniatis *et al.* (2001), supra). Suitable transfection methods include, without being limited thereto, e.g. electroporation techniques including modified electroporation techniques (e.g. nucleofection), calcium phosphate techniques, e.g. the calcium phosphate co-precipitation method, the DEAE-Dextran method, the lipofection method, e.g. the transferring mediated lipofection method, etc.. If the inventive method is to be applied *in vitro,* i.e. for enhanced expression of a protein of interest *in vitro,* any of the above disclosed host cells may be selected in optional step (2), preferably host cells which can be stably cultured *in vitro.* Furthermore, the transfected host cell as obtained by step (2) may be cultured in a suitable culture medium. The culture medium may be suitably selected by a person skilled in the art. Additionally, the culture medium may contain at least one (selection) marker, e.g. an antibiotic for antibiotic selection such as any of the antibiotics ampicillin, hygromycin, kanamycin, neomycin, and the like. However, a (selection) marker may be added only, if the inventive expression vector (A) and, optionally, the inventive expression vector (B), additionally carry the corresponding marker gene(s). If the inventive method is to be applied *in vivo,* i.e. for enhanced expression of a protein of interest *in vivo* in an animal or patient, preferably autologous cells may be used for the above purpose, i.e. cells which have been derived from the animal or patient to be treated itself/himself by isolation methods well known to a skilled person. Autologous cells typically do not trigger immune responses upon transplantation and are therefore highly advantageous for those *in vivo* methods.

Furthermore, the inventive *in vivo* or *in vitro* method for enhanced expression of a protein of interest comprises a step (3) of expressing the fusion protein and, generally, the protease, as defined herein. Expression is typically directed by the host cell used herein. However, if the inventive method is applied *in vitro,* expression of the fusion protein and, generally, the protease may be induced by selectively (a) inducing promoter(s) controlling expression of the fusion protein and, generally, the protease in inventive expression vectors (A) and, optionally, (B). The method of induction is dependent on the promoter used and may be appropriately chosen by a person skilled in the art upon general knowledge, e.g. by rising the temperature of the culture medium for temperature sensitive promoters, by adding IPTG (isopropyl-beta-D-thiogalactopyranoside) into the culture medium, etc. (see e.g. Maniatis *et al*., (2001), supra).

According to step (4) of the inventive *in vivo* or *in vitro* method for enhanced expression of a protein of interest the fusion protein and, generally, the protease are secreted, i.e. transported through the cellular membrane of the host cell. Secretion of the fusion protein occurs via its component (A1) as defined above, i.e. the secretory signal sequence. This secretory signal sequence may use the secretory pathway of the host cell, particularly, if the secretory signal sequence is derived from the specific host cell used for the inventive method. Secretion of the protease may be accomplished either by a fused secretory signal sequence as defined above or via secretory pathways of the host cell. During or subsequent to secretion, both the fusion protein and the protease as encoded by inventive expression vector (A) and, optionally, by inventive expression vector (B), are preferably located at or near the cell membrane and not released into the extracellular medium prior to proteolytic cleavage of the proteolytic cleavage site (A3) of the fusion protein.

According to step (5) of the inventive *in vivo* or *in vitro* method for enhanced expression of a protein of interest the fusion protein is proteolytically cleaved at the proteolytic cleavage site (A3) using a protease as defined above. Proteolytic cleavage preferably occurs during or subsequent to secretion of the fusion protein at the cell membrane of the host cell used. Due to proteolytic cleavage at the proteolytic cleavage site (A3), the protein of interest (A4) is released from its binding partners, e.g. the solubilizing agent (A2) and the secretory signal sequence (A1). Subsequent to proteolytic cleavage the protein of interest (A4) is typically located in the extracellular medium.

According to a further optional step (6) of the inventive *in vivo* or *in vitro* method for enhanced expression of a protein of interest, the (proteolytically cleaved and released) protein of interest (A4) may be purified *in vitro.* For purification any method known in the art may be used. Such methods include e.g. a variety of chromatographic procedures, such as ion exchange chromatography, affinity chromatography, gel chromatography or similar art recognized procedures (see e.g. Maniatis *et al.* (2001) *supra*). Chromatographic procedures, particularly affinity chromatographical procedures may suitably use specific tags for affinity purification as disclosed above (e.g. a hexahistidine-tag (his-tag, polyhistidine-tag), a streptavidine tag (strep-tag), a SBP-tag (a streptavidine binding tag), a GST(glutathione-S-transferase)-tag, etc.) Furthermore, purification may be carried out by means of an antibody epitope (an antibody binding tag), e.g. a myc-tag, a Swa11-epitope, a FLAG-tag, etc.), i.e. by recognition of said epitope by the corresponding (immobilized) antibody.

As a further embodiment, the present invention provides a method of treating a person suffering from a disease or disorder substantially caused by missing or defective expression of a protein of interest as defined above by using the inventive expression vector (A). Such diseases or disorders include e.g. anemia (erythropoietin), diabetes (insulin), hemophilia (coagulation factors VIII or IX), lactose intolerance (beta-lactamase), adenosine deaminase deficiency (adenosine deaminase), etc.. Furthermore, diseases or disorders include diseases or disorders caused by degenerative cell proliferation or aberrant cellular activity, e.g. such as cancer, allergy, autoimmune diseases, or infectious diseases, e.g. diseases caused by bacterial, viral, fungal or parasite pathogens.. Proteins of interest, which may be expressed by the inventive expression vector (A) include in this context any of the above proteins, preferably e.g. apoptotic proteins as disclosed above, bacterial or plant toxins (ribosome inactivating proteins), immunotoxins, vaccines (e.g. pathogen-derived antigens, tumor-associated antigens), proteins that stimulate or inhibit cell signaling (e.g. cytokines, antibodies, etc..), pathogen-specific antibodies, tumor-specific antibodies, etc..

The method of treatment for any of the above mentioned diseases or disorders preferably comprises the above defined steps of the inventive method for enhanced expression of a protein of interest *in vivo,* wherein the method additionally comprises a method step (2a) directed to administration of the transfected cells to a patient in need thereof. Particularly, the method of treatment may comprise following steps:
(1) optionally generating an inventive expression vector (A) encoding a fusion protein as defined above, and ,optionally, an inventive expression vector (B), encoding a protease as defined above;
(2) optionally transfecting a suitable host cell as defined above with the inventive expression vector (A) and, optionally, with the inventive expression vector (B), as generated i n step 1;
(2a) administering the transfected host cell(s) to a patient in need thereof;

In order to have the beneficial therapeutic effect in the patient to be treated, the following steps (3) to (5) will occur in the administered transfected host cells:
(3) expressing the fusion protein and, generally, the protease;
(4) secreting the fusion protein and, generally, the protease; and
(5) proteolytically cleaving the fusion protein at its proteolytic cleavage site using a protease according to steps (3) and (4) or as defined herein.

Method steps (1), (2), (3), (4) and (5) are preferably as defined above. Additionally, step (2a) is directed to administering the transfected host cells to a patient in need thereof. Typical administration forms in this context include, without being limited thereto, parenteral administration, e.g. by injection, for example, either subcutaneously, intradermally, subdermally, intramuscularly, or via intravenous, cutaneous or subcutaneous injection at the site of affliction. Other modes of administration, which may be suitable for treatment of any of the afore mentioned diseases or disorders, include transplantation of the transfected cells (preferably formulated in a suitable form, e.g. by addition of suitable pharmaceutical carriers, e.g. in the form of gels, capsules, tablettes, etc.). The cells transfected with inventive expression vector (A) and, optionally, with inventive expression vector (B), as defined above, may either be administered directly or via a composition comprising these cells. A composition for administration of cells transfected with at least one nucleic acid sequence carrying an inventive expression vector (A) for parenteral administration may, for example, be prepared as described in WO 03/002136, the disclosure of which is incorporated herein by reference. Typically, such a composition for administration of cells transfected with inventive expression vector (A) and, optionally, with inventive expression vector (B), as defined above, may be prepared as injectables either as liquid solutions or suspensions, preferably containing water (aqueous formulation) or may be emulsified. The term "aqueous formulation" is defined as a formulation comprising at least 50 % w/w water. Likewise, the term "aqueous solution" is defined as a solution comprising at least 50% w/w water, and the term "aqueous suspension" is defined as a suspension comprising at least 50 % w/w water.

For intravenous, cutaneous or subcutaneous injection, or injection at the site of affliction, the composition for administration of cells transfected with inventive expression vector (A) and, optionally, with inventive expression vector (B), as defined above, will be in the form of a parenterally acceptable aqueous solution which is pyrogen-free and has suitable pH, isotonicity and stability. Liquid compositions for administration of cells as defined above generally include a liquid vehicle such as water. Preferably, the liquid vehicle will include a physiological saline solution, dextrose ethanol or other saccharide solution or glycols such as ethylene glycol, propylene glycol or polyethylene glycol or combinations thereof may be included. Further examples include other isotonic vehicles such as physiological salt solutions, e.g. Ringer's solution or Lactated Ringer's solution.

It is possible that other ingredients may be present in a composition for administration of host cells transfected with inventive expression vector (A) and, optionally, with inventive expression vector (B), as defined above. Such additional ingredients may include wetting agents, emulsifiers, antioxidants, bulking agents, pH buffering agents (e.g. phosphate or citrate or maleate buffers), preservatives, surfactants, stabilizers, tonicity modifiers, chelating agents, metal ions, oleaginous vehicles, proteins (e.g. human serum albumin, gelatin or proteins) and/or a zwitterion (e.g. an amino acid such as betaine, taurine, arginine, glycine, lysine and histidine). Such ingredients are selected by a skilled person according to the specific requirements of the host cells transfected with inventive expression vector (A) and, optionally, with inventive expression vector (B), as defined above. With regard to buffers these are preferably selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethane, hepes, bicine, tricine, malic acid, succinate, maleic acid, fumaric acid, tartaric acid, aspartic acid or mixtures thereof. Each one of these specific buffers constitutes an alternative embodiment of the invention. The use of all of the afore-mentioned additives in a composition, such as the composition for administration of host cells transfected with inventive expression vector (A) and, optionally, with inventive expression vector (B), as defined above, is well-known to the skilled person, in particular with regard to concentration ranges of the same. For convenience reference is made to Remington: The Science and Practice of Pharmacy, 19th edition, 1995.

Host cells transfected in step (2) of the inventive method of treatment with inventive expression vector (A) and, optionally, with inventive expression vector (B), as defined above, or compositions comprising these cells are preferably administered in step (2a) of the above method in a manner compatible with the dosage formulation, and in such an amount as will be therapeutically effective. The quantity of transfected host cells to be administered, depends on the subject and the disease to be treated, including, e.g., the severity of the patient's disease. Suitable dosage ranges depend on the amount of biologically active protein of interest (a4), obtained after proteolytic cleavage of the fusion protein, during a predetermined time period and typically range in the order of one to several hundred micrograms per day (even though higher amounts in the range of 1-100 mg are also contemplated).

A further embodiment of the present invention is directed to a kit comprising an inventive expression vector (A) and, optionally, the inventive expression vector (B), for enhanced expression of a protein of interest as defined above. The kit may contain the inventive expression vector (A), wherein the nucleic acid sequence encoding the protein of interest (A4) is substituted by a nucleic acid sequence containing a multiple cloning site, suitable for inserting a nucleic acid sequence encoding a protein of interest as defined above. Multiple cloning sites are well known to a skilled person and may be obtained readily from the art as suitable. This substitution allows to adapt the inventive expression vector (A) to any protein of interest (A4). The kit may furthermore contain the inventive expression vector (A) in a suitable host cell as defined herein. Additionally, the kit may contain a manual or instruction guidelines, intended to explain the application of the kit to a skilled person. The kit may be used for enhanced expression of a protein of interest (A4) *in vitro*, or, if applicable, *in vivo.*

Finally, the inventive expression vector (A), optionally in combination with the inventive expression vector (B), as defined above as well as the inventive kit as defined herein may be used for the treatment or amelioration of a disease or a disorder as defined herein.

The invention is illustrated further in the accompanying Figures and Examples. However, it is not intended to limit the scope of the invention to the content of these Figures and Examples as shown in the following. The disclosure of any references as mentioned in the description is incorporated herein by reference.

### Figures:

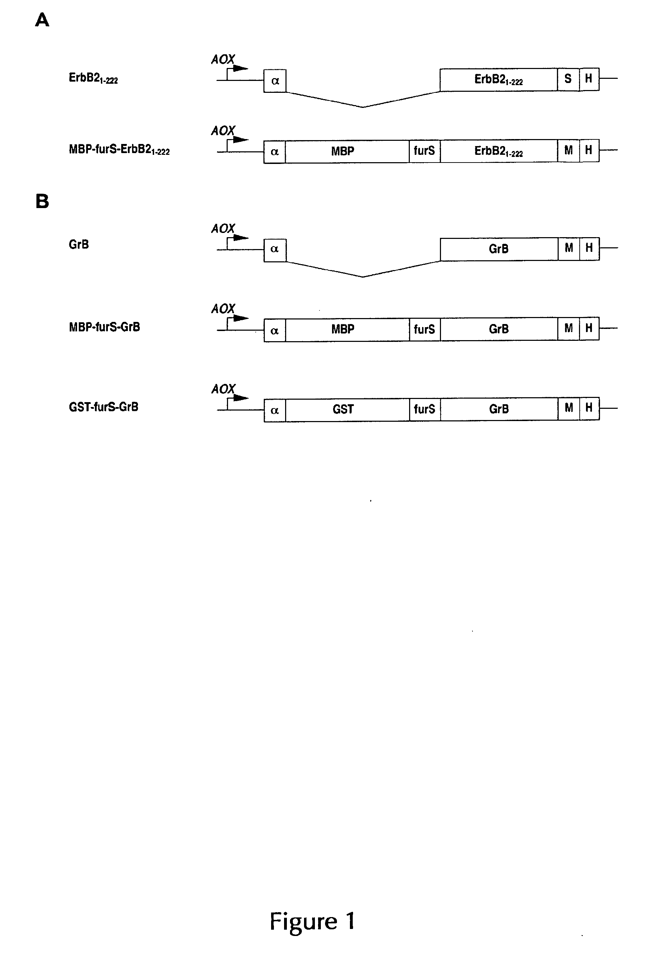

- **Figure 1:**: **shows a schematic representation of expression constructs**. Constructs for expression of human granzyme B (GrB) (amino acids 21-247) (*B*) and the N-terminal fragment of human ErbB2 (amino acids 1-222, ErbB2₁₋₂₂₂) in the yeast *Pichia pastoris (A).* GrB or ErbB2₁₋₂₂₂ are either expressed as non-fused proteins or as fusions with the *E. coli* maltose-binding protein (MBP) or the *Schistosoma japonicum* glutathione-*S*-transferase (GST). All expression constructs contain the N-terminal α-factor signal peptide to allow secretion of the recombinant proteins in yeast culture supernatants. The furin recognition motifs fur (Arg-Ala-Arg-Tyr-Lys-Arg) and furS (Arg-Ala-Arg-Tyr-Lys-Arg-Ser) are located between the MBP or GST component and the GrB or ErbB2₁₋₂₂₂ component to allow processing of the fusion proteins within the yeast secretory pathway in living cells. At the C-terminal ends, all constructs moreover contain a hexahistidine-tag (H) and a myc-tag (M) or a Swa11-epitope (S).

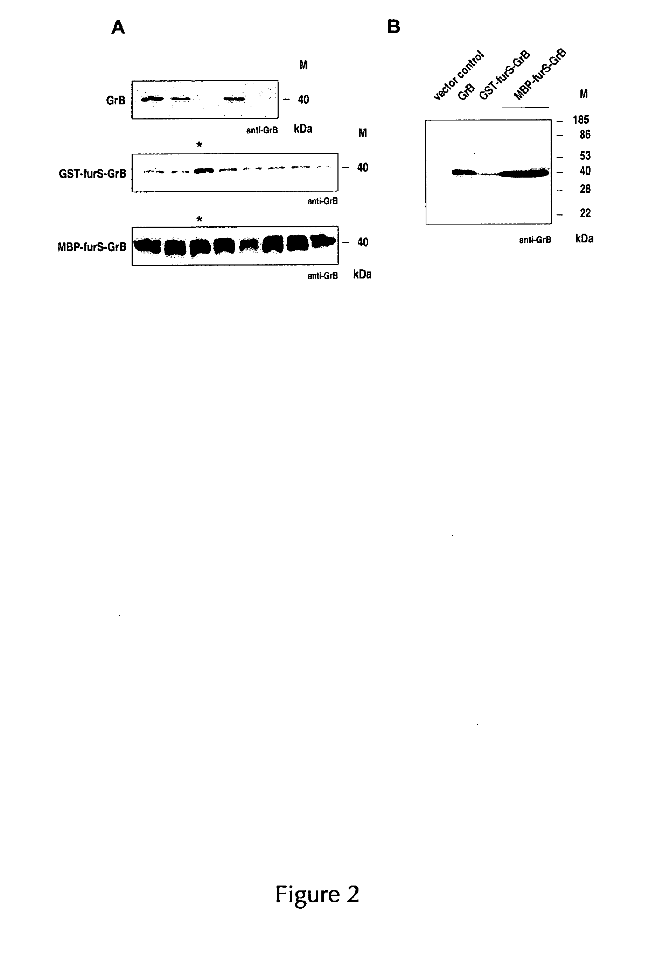

- **Figure 2:**: **depicts an immunoblot analysis of yeast supernatants derived from different expression clones.** (*A*) Different *Pichia pastoris* expression clones, transformed with pPIC9-GrB, pPIC9-GST-furS-GrB or pPIC9-MBP-furS-GrB were analyzed for expression and secretion of GrB. Each lane represents one clone. Supernatants of expression cultures were probed with the GrB specific mab 2C5 (*A*+*B*). Clones marked by an asterisk were used for further experiments. (*B*) Comparison of GrB expression levels of yeast clones transformed with pPIC9-GrB, pPIC9-GST-furS-GrB or pPIC9-MBP-furS-GrB respectively. The supernatant of a clone transformed with the empty pPIC9 vector served as a control.

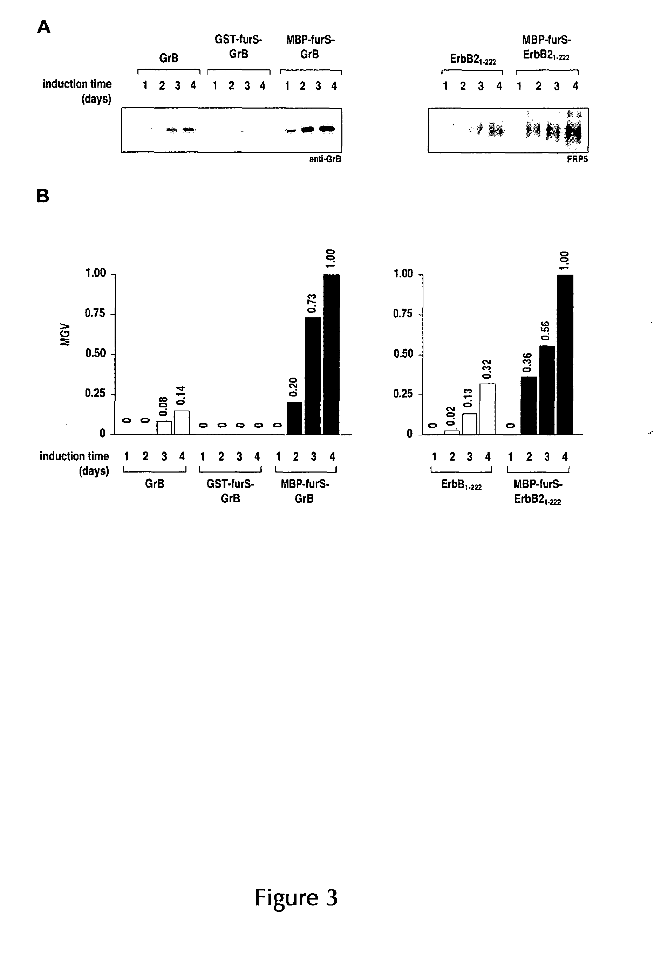

- **Figure 3:**: **illustrates a comparative quantification of GrB (left) and ErbB2₁₋₂₂₂ expression (right)**. Supernatants of expression cultures from yeast clones transformed with pPIC9-GrB, pPIC9-GST-furS-GrB, pPIC9-MBP-furS-GrB, pPIC9-ErbB2₁₋₂₂₂ or pPIC9-MBP-furS-ErbB2₁₋₂₂₂ were taken every 24 h and analyzed by immunoblotting using the GrB specific mab 2C5 or the ErbB2 specific mab FRP5 (*A*). Detected bands of immunoblots that were exposed in a way that the highest signal intensity reached saturation, were quantified with ImageJ 1.32 (NCBI) by measuring mean grey values. These values were calculated relative to the values obtained with the MBP-furS fusion proteins, that showed the highest expression levels (set to 1) (*B*).

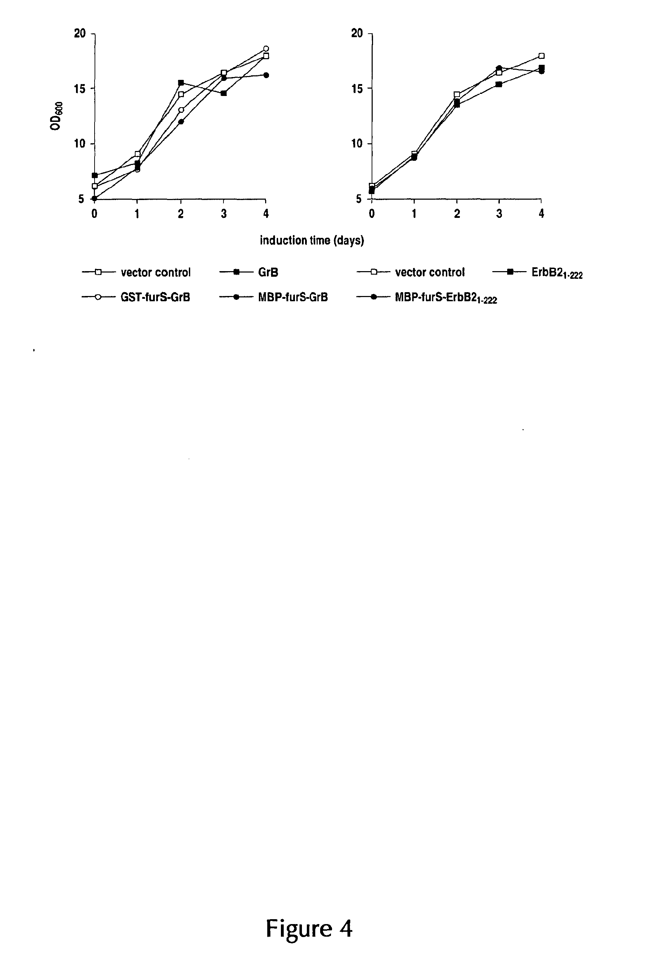

- **Figure 4:**: **depicts growth kinetics of expression clones**. Growth kinetics of yeast clones transformed with the GrB (*A*) or ErbB2 (*B*) expression constructs were monitored. Samples of expression cultures were taken every 24 h and the OD₆₀₀ was measured. A clone transformed with the empty vector pPIC9 was used as a control.

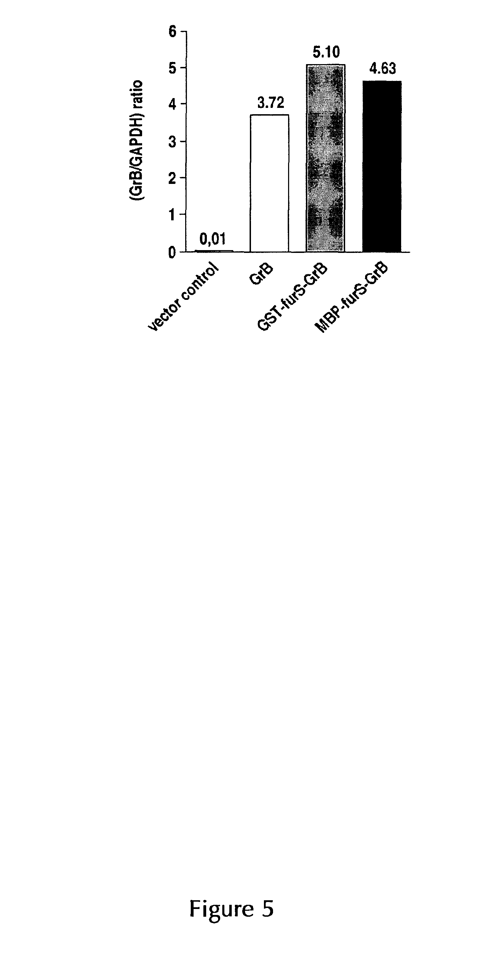

- **Figure 5:**: **illustrates the quantification of GrB mRNA levels transcribed by expression clones.** GrB mRNA levels of yeast expression clones transformed with the GrB expression constructs were measured in relation to GAPDH mRNA levels. RNA of yeast expression cultures (induced for 1 day) was isolated, cDNA was synthesized and analyzed by TaqMan PCR. Absolute copy numbers of GrB and GAPDH mRNA were determined by simultaneously analyzing samples containing plasmid standard titrations with defined copy numbers of GrB and GAPDH.

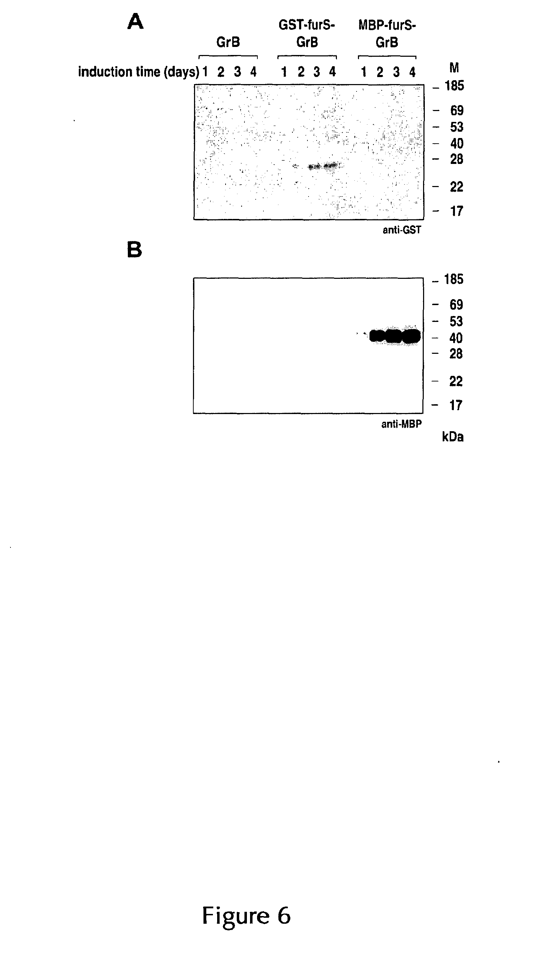

- **Figure 6:**: **shows an immunoblot analysis of yeast supernatants derived from GrB expression clones**. Secretion of the GST and MBP protein components of yeast expression clones transformed with GrB expression constructs was analyzed in immunoblots. The same supernatants as shown in figure 3 were used. GST (A) and MBP (B) were detected, using specific mabs against these proteins.

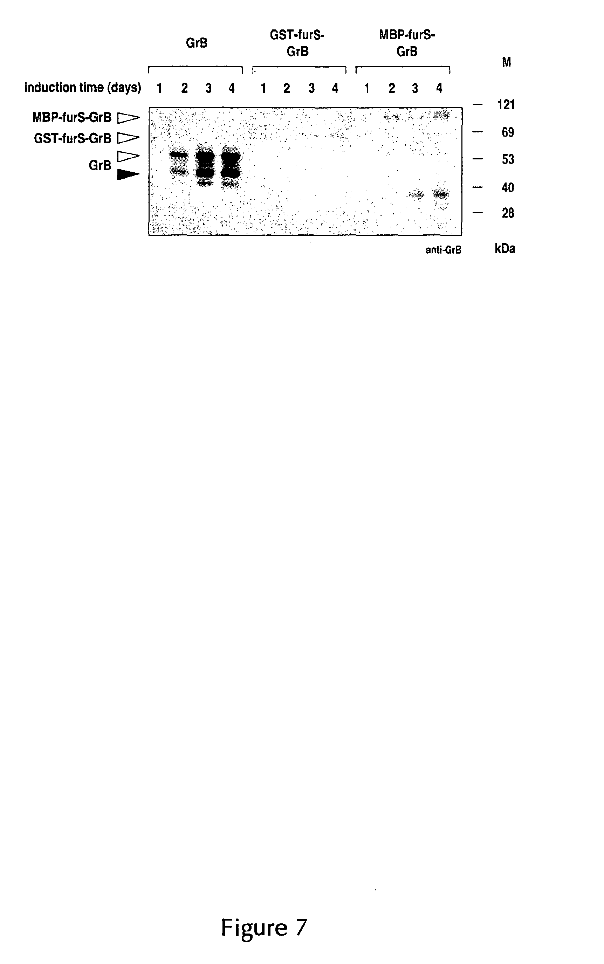

- **Figure 7:**: **shows an Immunoblot analysis of the intracellular fractions of GrB expression clones**. To detect accumulation of GrB in the intracellular fraction of yeast expression clones, immunoblot analysis of yeast lysates using a GrB specific mab were performed. For this analysis, samples from the same expression cultures as used for experiments shown in figure 3 and figure 6 were used. Bands corresponding to GrB, GST-furS-GrB and MBP-furS-GrB are marked by arrows.

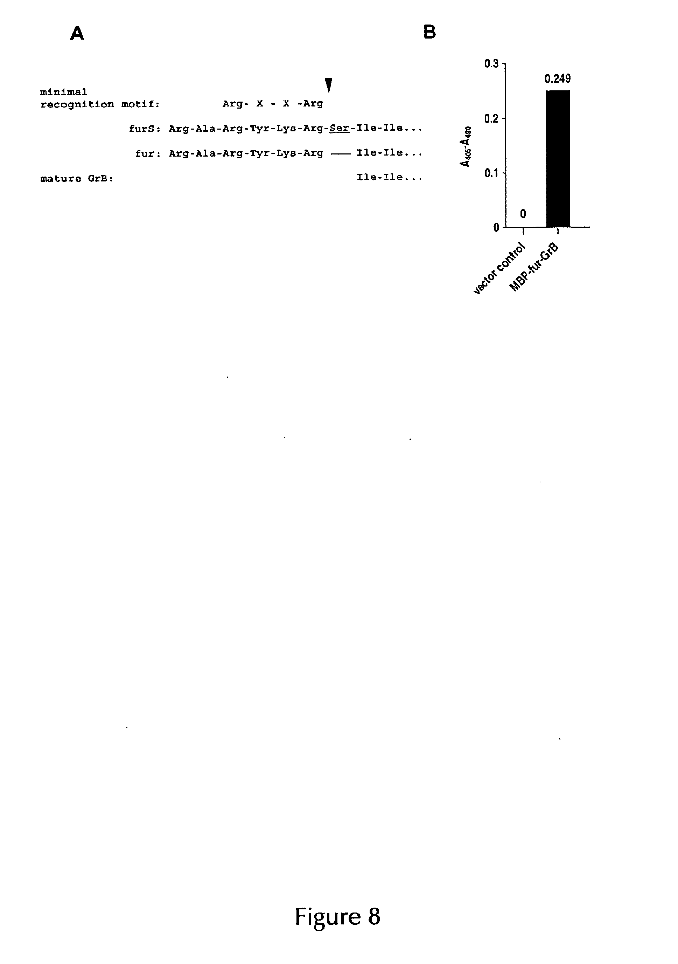

- **Figure 8:**: **depicts an analysis of enzymatic activity of GrB fusion proteins**. Culture supernatants from *Pichia* expression clones transformed with pPIC9-MBP-fur-GrB were analyzed by Western blotting with the mab 2C5. Supernatants corresponding to clones were further checked in enzymatic activity assays. (A) Amino acid sequence of furin cleavage sites. The furin recognition motifs fur and furS that are present in the MBP-fur-GrB and MBP-furS-GrB expression constructs are shown. The sequence of mature GrB starts with Ile-Ile. The theoretical cleavage site in which the proteins are processed by the furin homolog kexin are marked by an arrow. In the underlying experiment the fur-cleavage site was used. (*B*) The enzymatic GrB activity in supernatants of MBP-fur-GrB expression clones was determined in a colorimetric peptide cleavage assay. Supernatant of a clone transformed with the empty pPIC9 vector served as a control.

### Examples:

### 1. Construction of the yeast expression plasmids

To generate plasmids for the expression of the N-terminal part of human ErbB2 (ErbB2₁₋₂₂₂), we amplified the sequence coding for the maltose-binding protein (MBP) with the oligonucleotide primer 5'*Nde*I-MBP (AAAAAAACATATGATGAAAACTGAAGAAGGTAAACTGG) (SEQ ID NO: 6) and the 3' primer 3' *Xho*I*-Sal*I*-*furS*-*MBP (TTTTTCTCGAGTTTTTGTCGACGCTA CGTTTGTAACGAGCACGGCTCGAATTAGTCTGCGCGTCTTTCAGGGC) (SEQ ID NO: 7). The 3' primer added the sequence coding for an optimized furin recognition motif Arg-Ala-Arg-Tyr-Lys-Arg-Ser (SEQ ID NO: 5). In this reaction, the plasmid pMAL-NN-EGFP that contains the MBP sequence of the commercially available plasmid pMALc (New England Biolabs) was used as template (Bach et al., J Mol Biol 2001, 312:79-93). The MBP-furS fragment was subcloned as *Nde*I *Xho*I fragment in the respective cloning sites of a pBluescript II KS (Stratagene) derivative with modified polylinker (pBIIKS-MH) [Gerstmayer, 1997 #22], resulting in the plasmid pBIIKS-MBP-furS. The sequence coding for the N-terminal part of human ErbB2 (amino acids 1-222, ErbB2₁₋₂₂₂) followed by an epitope tag recognized by the mab 9E10 (myc-tag, M) and a polyhistidine-tag (H) was derived from the plasmid pSW5-ErbB2₁₋₂₂₂ (Rohrbach et al., J Immunol 2005, 174(9):5481-9) as *Sal*I *Xho*I fragment and was cloned to the 3' end of the sequence coding for MBP-furS in the plasmid pBIIKS-MBP-furS. From this plasmid, the sequence coding for MBP-furS-ErbB2₁₋₂₂₂-MH was obtained by digestion with *Avr*II and *Not*I and the fragment was cloned into the respective restriction sites of the yeast expression vector pPIC9 (Invitrogen, Karlsruhe, Germany) resulting in the plasmid pPIC9-MBP-furS-ErbB2₁₋₂₂₂. The yeast expression plasmid pPIC9-ErbB2₁₋₂₂₂ was created by inserting an ErbB21-m fragment digested with *Sal*I and *Xba*I into the respective cloning sites of pBIIKS-SH, resulting in the plasmid pBIIKS-ErbB2₁₋₂₂₂-SH, that codes for soluble ErbB2 followed by an epitope tag recognized by the mab Swa11 (S) and a hexahistidin-tag (H). From this plasmid, the ErbB2₁₋₂₂₂-SH fragment was obtained by digestion with *Avr*II and *Not*I and pPIC9-ErbB2₁₋₂₂₂ was generated by inserting this fragment into the corresponding cloning sites of pPIC9.

To construct the plasmids for expression of GrB in the yeast *Pichia pastoris,* fur-GrB-MH and furS-GrB-MH PCR fragments were prepared using the 5' oligonucleotide primers 5'Xbal-furS-GrB (ATATCTAGAACGTGCTCGTTACAAACGT AGCATC) (SEQ ID NO: 8) and 5' *Xba*I-fur-GrB (ATATCTAGAACGTGCTCGTTACA AACGTATCATCGG) (SEQ ID NO: 9) respectively, the 3' primer 3'*Xho*I-H (TTTGAT CTCGAGTCAGTGATGGTGATGATGGTG) (SEQ ID NO: 10) and the plasmid pPIC9-GrB as template (Giesubel et al., Biochem J 2006 394:563-73). Both PCR fragments differ in the sequence coding for the furin recognition motif (see results section for details). The amplified sequences were digested with *Xba*I and *Xho*I and inserted into the respective cloning sites of pBIIKS-MH. The resulting plasmids were referred to as pBIIKS-fur-GrB-MH and pBIIKS-furS-GrB-MH. Fragments encoding MBP or GST were obtained by PCR using the oligonucleotide primer pairs 5'*Nde*l-MBP/3'*Xba*l-MBP (AAATCTAGAGC CGAGCTCGAATTAGTCGCGCGTC) (SEQ ID NO: 11) and 5'Ndel-GST (AAAAAAACATATGATGTCCCCTATACTAGGT TATTGGAAAATTAAG) (SEQ ID NO: 12)/3'Xbal-GST (AAATCTAGAGCATCC GATTTTGGAGGATGGTCGCCACCACCAAACGTG) (SEQ ID NO: 13) respectively (5' primers are described above). The templates used in these reactions were the plasmids pMAL-NN-EGFP (see above) and pGEX-4T-1 (Amersham Biosciences). This plasmid contains the sequence of *Schistosoma japonicum* glutathione-*S*-transferase (GST) (Smith and Johnson, Gene 1988, 67:31-40). The MBP and GST fragments were inserted into the *Nde*I *Xba*I restriction sites of the plasmids pBIIKS-fur-GrB-MH and pBIIKS-furS-GrB-MH. From these plasmids, MBP-fur-GrB-MH, MBP-furS-GrB-MH and GST-furS-GrB-MH fragments were obtained by digestion with *Avr*II and *Not*I and the fragments were inserted into the respective restriction sites of pPIC9, resulting in the yeast expression vectors pPIC9-MBP-fur-GrB, pPIC9-MBP-furS-GrB and pPIC9-GST-furS-GrB.

### 2. Analysis of expression levels

For the expression of recombinant proteins in yeast, the *Pichia pastoris* expression system (Invitrogen) was used. *Pichia pastoris* GS115 cells were transformed with the linearized expression plasmids via electroporation and clones were selected on minimal dextrose (MD) plates according to the manufacturer's instructions. Transformation of the expression constructs led to stable genomic integration by homologous recombination, primarily into the yeast *AOX1* locus. Thus, expression of the gene of interest is controlled in these exemplary constructs by the methanol inducible alcohol oxidase I *(AOX1) promoter,* and the gene product is secreted into the culture supernatant due to the vector encoded secretion signal of the yeast α-factor.

For each construct, protein expression of several clones was analyzed by Western blotting. 10 ml buffered glycerol-complex medium (BMGY, pH 8) were inoculated with clones from freshly streaked MD plates and the cultures were grown at 28 °C for 2 days. Cells were spun down and the medium was exchanged for buffered methanol-complex medium (BMMY, pH 8) to induce expression of the *AOX1 promoter* controlled heterologous proteins.

The cultures were incubated for further 3 to 4 days at 28 °C in the presence of 2 % MeOH which was added every 24 h. The yeast cells were removed by centrifugation and the supernatants containing the recombinant proteins were analyzed by SDS-PAGE and subsequent Western blotting using the monoclonal antibody 2C5 (Santa Cruz) for the detection of GrB and FRP5 (Harwerth et al., J Biol Chem 1992; 267:15160-7) for the detection of ErbB2.

Quantitative analysis of protein expression was carried out by growing single colonies from a freshly streaked MD plate in 10 ml BMGY (pH 8) at 28 °C for 2 days. The cultures were diluted in 25 ml BMMY to an OD₆₀₀ of about 6 and were incubated for further 4 days at 28 °C in the presence of 2 % MeOH which was added every 24 h. Growth of the yeast cultures was monitored by measuring the OD₆₀₀ and samples were collected every 24 h. These samples were centrifuged to separate the recombinant protein containing supernatants from the yeast cells (intracellular fraction). The yeast cells were resuspended in PBS and both, the supernatant as well as the yeast intracellular fractions were analyzed by SDS-PAGE and subsequent Western blotting. The detected bands were quantified with ImageJ 1.32j (NCBI) by measuring mean gray values. These values were calculated relative to the values obtained with the MBP expression enhancement system (set to 1).

### 3. Isolation of yeast RNA and quantitative PCR

The RNA of yeast expression cultures (induced with 2 % MeOH for 1 day) was isolated to measure GrB mRNA levels by TaqMan real-time PCR. 1 ml samples of these cultures, each adjusted to an OD₆₀₀ of 8, were centrifuged for 5 min at 3000 g and the supernatants were discarded. Cell pellets were resuspended in buffer containing 1 M sorbitol, 0.1 M EDTA pH 7.4, 0.1 % mercaptoethanol and 5 µl lyticase (corresponding to 50 units, Sigma) and incubated for 30 min at 30 °C with moderate agitation. The spheroblasts were collected by centrifugation for 5 min at 300 g. Total RNA from yeast spheroblasts was isolated using the RNeasy Mini Kit (Qiagen) according to the manufacturer's instructions.

The GAPDH-fragment of *Pichia pastoris,* that was used for normalization in quantitative PCR analysis, was amplified by reverse transcription (RT) PCR using the Qiagen RT-PCR Kit according to the manufacturer's instructions, the primers GAPDH-Pichia-forward (GGCTATCACTGTCGGTATTAACGG) (SEQ ID NO: 14) and GAPDH-Pichia-reverse (GTGGAGTAACCGTACTCGTTGTCG) (SEQ ID NO: 15) and RNA isolated from *Pichia pastoris* as template. Using these primers, nucleotides 3-962 of *Pichia pastoris* GAPDH (GenBank Accession No. U62648) were amplified and the fragment was cloned into the plasmid pCR2.1 by TA cloning (invitrogen).

For quantitative PCR analysis, cDNA was synthesized from 1 to 5 µg of yeast RNA, according to a standard protocol (van Dongen et al., Leukemia 1999; 13:1901-28). Plasmid standard titrations with defined copy numbers of GrB and GAPDH as housekeeping gene were analyzed simultaneously with the yeast samples. TaqMan PCR were performed in duplicate reactions on an Abi PRISM 7700 sequence detection system (Applied Biosystems, Weiterstadt, Germany) using standard conditions (50 °C for 2 min, 95 °C for 10 min, 45 cycles at 95 °C for 15 sec, 60 °C for 1 min). To amplify the GrB fragment, 2.5 µl of template was used in 25 µl reaction mixtures consisting of the GrB-forward (TGGAGGCCCTCTTGTGTGTAA) (SEQ ID NO: 16) and GrB-reverse (GCATGCCATTGTTTCGTCC) (SEQ ID NO: 17) primers and the GrB specific probe GrB-so (AAGGTGGCCCAGGGCATTGTCTC) (SEQ ID NO: 18), labeled with FAM at the 5' end and TAMRA at the 3' end. To amplify GAPDH for normalization, GAPDH-forward (GTCAACGACACTTTCGGAATTG) (SEQ ID NO: 19) and GAPDH-reverse (TTGAGTGGCGGTCATGGA) (SEQ ID NO: 20) primers were used in combination with the GAPDH specific probe GAPDH-so (TCCGGTTTGATGACCACCGTCCA) (SEQ ID NO: 21) carrying the same labels. All primers and probes were used at a final concentration of 200 nM, other reagents were used as recommended by the suppliers (Platinum RTS qPCR-Super-Mix-UDG, Rox, Invitrogen).

### 4. Results

### 4.1 Analysis of expression of fusion proteins (A4)

### 4.1.1 Constructs (see also above)

For analysis of expression we have designed expression constructs of GrB and the N-terminal part of human ErbB2 (ErbB2₁₋₂₂₂) in the yeast *Pichia pastoris* (see above) These constructs consist of the sequence coding for the N-terminal α-factor secretion signal followed by the maltose-binding protein, the optimized furin recognition motif and either the sequence coding for GrB or for ErbB2_{1-222·} Alternatively, we have used the sequence coding for GST instead of that coding for MBP.

Yeast clones transformed with these constructs were used to analyze whether the solubility and stability enhancing properties of MBP and GST in the context of fusion proteins are conferrable to proteins expressed in the yeast *Pichia pastoris*. The furin recognition motif between MBP or GST and the GrB or ErbB2₁₋₂₂₂ components should be processed within the yeast secretory pathway, leading to secretion of non-fused GrB or ErbB2₁₋₂₂₂ in culture supernatants.

### 4.1.2 Qualitative expression analysis

For expression analysis of GrB, we have used two different furin recognition motifs Arg-Ala-Arg-Tyr-Lys-Arg (SEQ ID NO: 4) and Arg-Ala-Arg-Tyr-Lys-Arg-Ser (SEQ ID NO: 5). The terminal serine (underlined) occurs in many physiological substrates of furin and could therefore make the recognition motif more efficiently cleaved by the protease. However, said serine may be omitted for proteins of interest allowing no N-terminal extension of their native protein sequence.

Two constructs using the unmodified pPIC9 expression vector, in which the proteins of interest are directly fused with the α-factor secretion signal pPIC9-GrB (Giesubel, 2006, supra) and pPIC9-ErbB2₁₋₂₂₂ were used as references. All constructs are shown in detail in figure 1.

Expression of GrB or ErbB2₁₋₂₂₂ by yeast clones was analyzed by SDS-PAGE with supernatants from yeast cultures that were induced for 4 days and subsequent Western blotting using the GrB specific mab 2C5 (figure 2A) or the anti-ErbB2 mab FRP5 (data not shown). For each construct, several clones were tested and one clone that showed a representative expression level was chosen for further experiments (marked by an asterisk). As reference clone for the expression of GrB using the unmodified pPIC9 expression system, we used an established clone that had previously shown high expression levels (Giesubel, 2006, supra).

Each clone was used to inoculate expression cultures in a larger scale as described above. The cultures were induced for 3 days in the presence of 2 % MeOH. Culture supernatants were cleared by centrifugation, separated by SDS-PAGE and analyzed by Western blotting using the GrB specific mab 2C5 (figure 2B). All proteins migrated in single bands at the expected molecular weight of 40 kDa, indicating that both fusion proteins (GST-furS-GrB and MBP-furS-GrB) are completely processed, presumably within the furin recognition motif. The calculated molecular weights of the full-length fusion proteins are 71 kDa for MBP-furS-GrB, 56 kDa for GST-furS-GrB and 29 kDa for non-fused GrB. However, GrB expressed in *Pichia pastoris* is a glycosylated protein with an apparent molecular weight of 40 kDa (Giesubel, 2006, supra). All constructs migrated at the expected molecular weight of non-fused glycosylated GrB.

The expression level of GrB expressed as MBP-furS-GrB fusion protein in yeast supernatants was significantly higher than that obtained from the clone transformed with pPIC9-GrB (see figure 2B). GrB produced as fusion protein by the expression clone transformed with pPIC9-GST-furS-GrB showed very low expression levels compared to the other constructs. To quantify the effect of the MBP-furS component on GrB and ErbB2₁₋₂₂₂ protein yields, samples of expression cultures were taken every 24 h. These samples were cleared by centrifugation and analyzed by SDS-PAGE and subsequent Western blotting as described (figure 3A). To compare the amount of GrB and ErbB2₁₋₂₂₂ present in these supernatants, the bands of a Western blot were quantified using the program ImageJ. The mean grey values thus obtained are depicted in histograms (figure 3B). Western blots used for these quantifications were exposed in a way that the highest signal intensity reached saturation. The yields of GrB and ErbB2₁₋₂₂₂ expressed as MBP-furS fusion proteins both were significantly higher than the yields of the non-fused proteins (for GrB about 9 times higher at day 3 post induction and about 7 times higher at day 4 post induction, for ErbB2₁₋₂₂₂ about 4 times higher at day 3 post induction and about 3 times higher at day 4 post induction). GrB expressed as GST-furS fusion.protein again showed relatively weak expression levels. To exclude the effect of different growth kinetics of the transformed yeast clones on the amount of protein in culture supernatants, we measured the OD₆₀₀ of the cultures every 24 h. The results are depicted in figure 4. All cultures showed comparable growth rates, indicating that the increased expression levels were not due to differences in proliferation of the clones.

### 4.1.3 Quantitative expression analysis

The increased protein yields of the MBP-furS fusion proteins demonstrated that the MBP-furS component functioned as an expression enhancer in the context of these fusion proteins. We wanted to know if the MBP-furS component functioned on the transcriptional, translational or posttranslational level. Thus we analyzed GrB mRNA levels of the expression clones by quantitative PCR. Yeast RNA was isolated from expression cultures that were induced for 1 day in the presence of 2 % MeOH. The cDNAs were prepared and TaqMan PCR were performed in duplicate reactions on an Abi PRISM 7700 sequence detection system.

Plasmid standard titrations with defined copy numbers of GrB and GAPDH were analyzed simultaneously with the yeast samples. The GrB/GAPDH mRNA ratios are shown in figure 5. Both clones transformed with the GrB fusion constructs (pPIC9-MBP-furS-GrB and pPIC9-GST-furS-GrB) showed slightly increased GrB mRNA levels compared to the clone transformed with pPIC9-GrB. The pPIC9-GST-furS-GrB clone showed the highest GrB/GAPDH mRNA ratio but the lowest GrB expression levels (see figure 3A). Taken together, we did not find a correlation between the observed expression levels of GrB and different mRNA levels. This indicated that the MBP-furS component acted on the translational or posttranslational level.

When analyzing the yeast supernatants by Western blotting with antibodies detecting the MBP or GST components of the fusion proteins, bands migrating at 40 kDa for MBP and 25 kDa for GST could be detected (figure 6B and 6A). These molecular weights correspond to the calculated molecular weights of the MBP (40.6 kDa) and GST (25.6 kDa) moieties, indicating that both protein components were secreted. Using the MBP and GST specific mabs, full-length GrB fusion proteins in yeast supernatants could not be detected (figure 6A and 6B), confirming the results obtained with the GrB specific mab (figure 2A). This demonstrates that both, the MBP-furS-GrB and the GST-furS-GrB fusion proteins were completely processed, presumably within the furin recognition motif.

As described before, MBP was referred to as a chaperone in the context of fusion proteins expressed in *E*. *coli*. If this chaperone activity of MBP also exists for the expression of MBP fusion proteins in the yeast *Pichia pastoris*, higher levels of soluble heterologous proteins in yeast supernatants compared to proteins expressed in the non-fused form are expected. This is what we have detected for both proteins GrB and ErbB2₁₋₂₂₂. Proteins that are secreted by the exocytic pathway are translocated into the membrane or lumen of the ER and are then transported to the Golgi apparatus where sorting to cellular locations occurs. For successful vesicular transport, proteins need to be properly folded. The surveillance system that detects improper folding has been referred to as "ER quality control" and is present in all eukaryotes, including yeast (Ellgaard and Helenius, Nat Rev Mol Cell Biol 2003, 4:181-91; Kostova and Wolf; Embo J 2003, 22:2309-17). Misfolded proteins are retained in the ER and are in certain cases sequestered. In some cases, proteins that cannot fold properly are retranslocated from the ER and subjected to ER-associated degradation by the ubiquitin-proteasome system. However, proteins that are highly overexpressed, like GrB expressed under the control of the strong *AOX1 promoter,* can accumulate in the ER when they are misfolded. To analyze accumulation of GrB in yeast cells, we separated the yeast intracellular fractions of expression cultures that were induced for 4 days by SDS-PAGE and detected intracellular GrB with GrB mab 2C5 by Western blotting (see figure 7). Two bands migrating at 44 kDa and 53 kDa probably corresponding to processed glycosylated GrB (band at 44 kDa) and the non-processed form of glycosylated GrB (GrB, band at 53 kDa) could be detected in cell lysates of yeast cells that express non-fused GrB. Accumulation of GrB expressed as MBP-furS or GST-furS fusion proteins did not occur. This indicates that the MBP component promotes proper folding of the fusion partner GrB as described for the expression of MBP fusion proteins in *E. coli,* and as a consequence, the protein is secreted more efficiently.

### 4.2 Analysis of enzymatic activity of exemplary inventive proteins of interest

### 4.2.1 Enzymatic activity of GrB derivatives

To detect enzymatic activity of the recombinant GrB proteins in yeast supernatants as measure for native protein conformations, the synthetic tetrapeptide Ac-IETD-pNA (Acetyl-Ile-Glu-Thr-Asp-p-Nitroanilide, Alexis) was used as a synthetic substrate for GrB in colorimetric peptide cleavage assays. Processing of the peptide by GrB leads to release of p-nitroaniline, which gives rise to colour development. *In vitro* cleavage reactions were set up in 96 well plates in a total volume of 100 µl each, containing 200 µM Ac-IETD-pNA (stock: 20 mM in DMSO) in reaction buffer (10 mM HEPES pH 7.4, 140 mM NaCl, 2.5 mM CaCl₂) and 10 µl of GrB containing yeast supernatants from cultures that were induced for 4 days. The samples were incubated at 37 °C for 1 h. Colour development due to cleavage of the substrate was quantified by reading the absorbances at 405 nm and 490 nm with a microplate reader (Molecular Devices Spectra MAX 340). Peptide substrate incubated in reaction buffer without protein served as blank. The yeast supernatant of a clone transformed with the empty vector pPIC9 served as a control.

Culture supernatants of expression clones transformed with MBP-fur-GrB were analyzed for GrB expression by Western blotting. Moreover we checked for GrB catalytic activity in supernatants of one clone for the expression construct (figure 8 B). An average enzymatic activity of 0,249 at A₄₀₅-A₄₉₀ was detectable for the MBP-fur-GrB expression construct.

### Advantages

We have developed a novel expression vector (A) (optionally in combination with an expression vector (B)) for the enhanced expression of a protein of interest that allow to significantly enhance solubility and expression yield but do not require any further manipulation of expressed protein of interest, e.g. proteolytic cleavage subsequent to a first purification of a precursor fusion protein. For this purpose, the expression vector (A) utilizes a fusion protein encoding a solubilizing agent, such as the maltose binding protein, and a protein of interest, which are fused via a proteolytic cleavage site. Advantageously, the inventive fusion protein gets expressed in the host cell and processed using an heterogenously or endogenously expressed protease during secretion to the culture supernatant.

As a particular example, we have fused MBP as a solubilizing agent to the N-terminus of two prototype molecules, mature GrB and an N-terminal part of the growth factor receptor ErbB2 (ErbB2₁₋₂₂₂). It has been described for bacterially expressed recombinant proteins that MBP enhances the yield, stability and solubility of proteins fused to it, thereby acting like a chaperon in the context of fusion proteins. In our experiments we could show that these properties of MBP are conferrable to secreted recombinant proteins (proteins of interest) expressed in an established host cell, e.g. *Pichia pastoris.* Expression levels of GrB and ErbB2₁₋₂₂₂ could be enhanced by MBP fusion. Protein yields could be increased up to nine fold for GrB and up to four fold for ErbB2₁₋₂₂₂, each in comparison to the reference proteins expressed in the non-fused form. The increased production yield of MBP-furS-GrB or MBP-fur-GrB is not explainable with different growth kinetics of the yeast clones, as they all showed the same proliferation rate as measured by the OD₆₀₀ of the expression cultures. By quantitative TaqMan PCR we could also demonstrate that the MBP component acts at the translational or posttranslational level, as expected. For secretion of production of MBP fusion proteins in *Pichia pastoris* we used the yeast mating type α-factor signal peptide of *Saccharomyces cerevisiae* that gets removed by the endogenous *Pichia* protease kexin (KEX2) within the yeast secretory pathway. With the two prototype molecules MBP-furS-GrB and MBP-furS-ErbB2₁₋₂₂₂ we could show that they get completely processed within the yeast secretory compartment, resulting in secretion of the protein of interest in its non-fused form. By using two different furin recognition motifs (furS/ fur) that only differ in the presence of the terminal serine we could show that proteolysis occurs at the expected site previous to the terminal serine and leads to functional proteins.

## Claims

1. Expression vector (A) for enhanced expression of a protein of interest, encoding a fusion protein comprising:
(A1) a secretory signal sequence;
(A2) a solubilizing agent;
(A3) a proteolytic cleavage site; and
(A4) a protein of interest;

2. Expression vector (A) according to claim 1, wherein the secretory signal sequence (A1) is selected from the group consisting of secretory signal sequences derived from a secreted cytokine, a clotting factor, an immunoglobulin, a secretory enzyme, secreted matrix metalloproteinases (MMP), including a stromelysin leader sequence, from secreted human alkaline phosphatase (SEAP), pro-exendin, including a proexendin-4 leader sequence, or are derived from pro-helodermin, pro-glucose-dependent insulinotropic polypeptide (GIP), pro-insulin-like growth factor (lGF1), preproglucagon, alpha-1 antitrypsin, insulin-like growth factor 1, human factor IX, human lymphotoxin A (Genbank Accession no. BAA00064), human clusterin (Genbank Accession no. AAP88927), neuroendocrine protein 7B2 precursor (secretory granule endocrine,protein I) (secretogranin V) (homo sapiens); accessory gland-specific peptide 95EF precursor (male accessory gland,secretory protein 316) (drosophila melanogaster); antileukoproteinase 1 precursor (ALP) (HUSI-1) (seminal proteinase, inhibitor) (secretory leukocyte protease inhibitor) (BLPI) (mucus, proteinase inhibitor) (MPI) (WAP four-disulfide core domain protein 4), (protease inhibitor WAP4 (homo sapiens); antileukoproteinase 1 precursor (ALP) (secretory leukocyte protease, inhibitor) (mus musculus); complement C1q tumor necrosis factor-related protein 3 precursor (secretory protein CORS26) (homo sapiens); small inducible cytokine A2 precursor (CCL2) (monocyte chemotactic, protein 1) (MCP-1) (monocyte chemoattractant protein-1) (monocyte, chemotactic and activating factor) (MCAF) (monocyte secretory protein, JE). (homo sapiens); CAMPATH-1 antigen precursor (CD52 antigen) (epididymal secretory, protein E5) (canis familiaris); CAMPATH-1 antigen precursor (CD52 antigen) (CDW52) (cambridge, pathology 1 antigen) (epididymal secretory protein E5) (homo sapiens); chitinase 3-like protein 3 precursor (secretory protein Ym1), (eosinophil chemotactic cytokine) (ECF-L) (mus musculus); and chromogranin A precursor (CgA) (pituitary secretory protein I) (SP-I) (bos taurus), pelB signal sequence of the *Erwinia carotovora* pectate lyase having the sequence MKYLLPTAAAGLLLLAAQPAM; ompA signal sequence of the *Escherichia coli* outer membrane protein A having the sequence MKKTAIAIAVALAGFATVAQ; ompF signal sequence of the *Escherichia coli* outer membrane protein F having the sequence MMKRNILAVIVPALLVAGTANA; ompT signal sequence of the *Escherichia coli* protease 7 having the sequence MRAKLLGIVLTTPIAISSFA; phoA signal sequence of the *Escherichia coli* PhoA protein having the sequence MKHSTIALALLPLLFTPVTKA; and lg kappa-chain leader sequence murine: lg kappa-chain V-J2-C leader sequence having the sequence METDTLLLWVLLLWVPGSTGD.

3. Expression vector (A) according to claim 1 or 2, wherein the solubilizing agent (A2) is selected from the group consisting of maltose-binding protein (MBP), glutathion-S-transferase (GST) thioredoxin (TRX), ubiquitin, protein A, DsbA, domain 1 of IF2 protein of *E. coli,* NusA and and chaperones and chaperone cofactors including GroES, GroEL, GrpE, DanK, DnaJ, Hsp100, Hsp90, Hsp70, Hsp60 and Hsp25.

4. Expression vector (A) according to any of claims 1 to 3, wherein the proteolytic cleavage (A3) site is selected from a peptide sequence comprising about 5 to 30 amino acids.

5. Expression vector (A) according to claim 4, wherein the proteolytic cleavage site (A3) is selected from the group consisting of proteolytic cleavage sites of endoproteases of the subtilisin family, including the proteases Kexin (KEX2) and Furin having proteolytic cleavage sites fitting the Arg-X-Lys/Arg-Arg motif (SEQ ID NO: 1), including Arg-X-Lys/Arg-Arg (SEQ ID NO: 2), Arg-Gln/Tyr-Lys/Arg-Arg (SEQ ID NO: 3), Arg-Ala-Arg-Tyr-Lys-Arg (SEQ ID NO: 4) and or Arg-Ala-Arg-Tyr-Lys-Arg-Ser (SEQ ID NO: 5), proteolytic cleavage sites of subtilisins including PC2, PC1/PC3, PACE4, PC4, PC5/PC6, LPC/PC7IPC8/SPC7 and SKI-1, the proteolytic cleavage site of chymotrypsin, the proteolytic cleavage sites of enterokinases, including the enterokinase recognition motifs Asp-Asp-Asp-Aps-Lys* and Asp/Glu-Arg-*Met, the proteolytic cleavage site of coagulation factor Xa, including the recognition motif Glu-Gly-Arg*, the proteolytic cleavage site of Thrombin, including the recognition motif Leu-Val-Pro-Arg*Gly-Ser, the proteolytic cleavage site of tobacco etch virus (TEV) protease, including the recognition motif Glu-Asn-Leu-Tyr-Phe-Gln*Gly/Ser, the proteolytic cleavage site of tobacco vein mottling virus (TVMV) protease, including the recognition motif Glu-Thr-Val-Arg-Phe-Gln*Gly/Ser, the proteolytic cleavage site of Granzyme B, including the recognition motif Ile-Glu-Pro-Asp* and the proteolytic cleavage site of Caspase-3, including the recognition motif Asp-Glu-Val-Asp*, as well as a sequence showing about 80%, more preferably 90% and most preferably 95 or even 99 % sequence identity with a proteolytic cleavage site (A3) as defined above.

6. Expression vector (A) according to any of claims 1 to 5, wherein the protein of interest (A4) is selected from the group consisting of growth hormones or growth factors, including TGFα and insuline like growth factors (IGFs), from molecules that effect metabolism and hematopoiesis including α-anti-trypsin, insulin, LDL-receptor, erythropoietin (EPO), GATA-1, molecules of the blood clotting system including factors VIII and XI, from enzymes including [beta]-galactosidase (lacZ), DNA restriction enzymes, DNA polymerases, lysozyme, proteases and protease inhibitors including papain, bromelain, keratinases, trypsin, chymotrypsin, pepsin, rennin (chymosin), suizym, nortase, from cytokines including TNFα, TNF-α receptor, IL-2, etc., antibodies, including monoclonal antibodies, single-chain antibodies (sc-antibodies), including scFv, anti-ErbB2 scFv(FRP5), pathogen-specific antibodies, tumor-specific antibodies, an apoptotic factor or an apoptosis related enzyme including AIF, Apaf e.g. Apaf-1, Apaf-2, Apaf-3, or APO-2 (L), APO-3 (L), Apopain, Bad, Bak, Bax, Bcl-2, Bcl-x_{L}, Bcl-x_{S}, bik, CAD, Calpain, Caspases, including Caspase-1, Caspase-2, Caspase-3, Caspase-4, Caspase-5, Caspase-6, Caspase-7, Caspase-8, Caspase-9, Caspase-10, Caspase-11, ced-3, ced-9, c-Jun, c-Myc, crm A, cytochrome C, CdR1, DcR1, DD, DED, DISC, DNA-PK_{CS}, DR3, DR4, DR5, FADD/MORT-1, FAK, Fas (Fas-ligand CD95/fas (receptor)), FLICE/MACH, FLIP, Fodrin, fos, G-Actin, Gas-2, Gelsolin, granzyme A/B, ICAD, ICE, JNK, Lamin A/B, MAP, MCL-1, Mdm-2, MEKK-1, MORT-1, NEDD, NF-_{κ}B, NuMa, p53, PAK-2, PARP, Perforin, PITSLRE, PKCδ, pRb, Presenilin, prICE, RAIDD, Ras, RIP, Sphingomyelinase, thymidine kinase from Herpes simplex, TRADD, TRAF2, TRAIL-R1, TRAIL-R2, TRAIL-R3, Transglutaminase, bacterial or plant toxins, immunotoxins, vaccines, or any fragment of the above proteins showing a sequence identity of at least 40%, 50%, 60%, 70%, 80%, 90%, 95% or 99% with their corresponding non-modified protein of interest (A4) as defined above.

7. Expression vector (A) according to any of claims 1 to 6, wherein the fusion protein further comprises at least one tag for purification (A5) of the protein of interest selected from the group consisting of a hexahistidine-tag (his-tag, polyhistidine-tag), a streptavidine tag (strep-tag), a SBP-tag (a streptavidine binding tag), a GST(glutathione-S-transferase)-tag, and an antibody epitope (an antibody binding tag), including a myc-tag, a Swa11-epitope, and a FLAG-tag.

8. Expression vector (A) according to any of claims 1 to 7, wherein the expression vector (A) encodes a fusion protein comprising from N- to C-terminus (A1) a secretory signal sequence; (A2) a solubilizing agent; (A3) a proteolytic cleavage site; (A4) a protein of interest; and optionally (A5) a tag for purification of the protein of interest.

9. Expression vector (A) according to any of claims 1 to 8, wherein the expression vector (A) furthermore encodes a protease (A6), capable of cleaving the proteolytic cleavage site (A3) subsequent to or during secretion of the fusion protein.

10. Expression vector (A) according to claim 9, wherein the protease (A6) is selected from the group consisting of bacterial and mammalian endoproteases of subtilisins and the subtilisin-like family including Kexin (KEX2) and Furin, or proteases selected from Coagulation factor Xa, Thrombin, enterokinase(s), tobacco etch virus (TEV) protease, tobacco vein mottling virus (TVMV) protease and Granzyme B, Caspase-3.

11. Expression vector (A) according to claim 9 to 10, wherein the protease (A6) is fused to an ER retention signal (KDEUHDEL), a transmembrane domain, or a secretory signal sequence as defined in claim 2.

12. Host cell transfected with an expression vector (A) according to any of claims 1 to 11.

13. Host cell according to claim 12, wherein the host cell is selected from the group consisting of prokaryotic host cells including *Escherichia coli,* including XL1-blue, *B. subtilis,* salmonella, pneumococcus, from algae, fungae, or from eukaryotic host cells including Saccharomyces *cerevisiae,* Schizosaccharomyces *pombe,* Pichia *pastoris,* including GS115, cells from mammalian hosts cells including PMBCs, lymphozytes, 293T (embryonic kidney cell line), HeLa (human cervix carcinoma cells) and host cells established for laboratory use including HEK293-, Sf9- and COS-cells, or from autologous cells.

14. Host cell according to claim 12, wherein expression vector (A) encodes a fusion protein comprising components (A1), (A2), (A3), (A4), and optionally (A5), and wherein the host cell furthermore comprises an expression vector (B) encoding a protease as defined according to any of claims 9 to 11.

15. Host cell according to claim 12, wherein expression vector (A) encodes a fusion protein comprising components (A1), (A2), (A3), (A4), and optionally (A5), and wherein the host cell endogenously encodes a protease as defined according to any of claims 9 to 11.

16. *In vitro* method for enhanced expression of a protein of interest comprising following steps:
(1) optionally generating an expression vector (A) according to any of claims 1 to 11, encoding a fusion protein, and, optionally, an expression vector (B) as defined in claim 14, encoding a protease;
(2) optionally transfecting a suitable host cell with the expression vector (A) and, optionally, with expression vector (B), as generated in step (1);
(3) expressing the fusion protein and, generally, the protease;
(4) secreting the fusion protein and, generally, the protease; and
(5) proteolytically cleaving the fusion protein at the proteolytic cleavage site (A3) using the protease according to steps (3) and (4) or as defined in any of claims 9 to 11.

17. *In vitro* method for enhanced expression of a protein of interest according to claim 16, wherein the suitable host cell is selected from prokaryotic or eukaryotic host cells, including bacterial, fungal, plant, human and animal host cells.

18. *In vitro* method for enhanced expression of a protein of interest according to claim 16 or 17, wherein the host cell is selected from the group consisting of prokaryotic host cells including *Escherichia coli,* including XL1-blue, *B. subtilis,* salmonella, pneumococcus, from algae, fungae, or from from eukaryotic host cells including Saccharomyces *cerevisiae,* Schizosaccharomyces *pombe,* Pichia *pastoris,* including GS115, cells from mammalian hosts cells including PMBCs, lymphozytes, 293T (embryonic kidney cell line), HeLa (human cervix carcinoma cells) and host cells established for laboratory use including HEK293-, Sf9-, CHO(chinese hamster ovary)- and COS-cells, or from autologous cells.

19. Kit comprising an expression vector (A) according to any of claims 1 to 11, and, optionally, an expression vector (B) as defined in claim 14, for enhanced expression of a protein of interest and optionally a manual or instruction guidelines.

20. Kit according to claim 19, wherein a nucleic acid sequence of expression vector (A) encoding the protein of interest (A4) is substituted by a nucleic acid sequence containing a multiple cloning site.

21. Kit according to claim 19 or 20, wherein the expression vector (A) and, optionally, expression vector (B) are transfected into a host cell as defined in any of claims to 12 to 15.

22. Use of an expression vector (A) according to any of claims 1 to 11, optionally in combination with an expression vector (B) as defined in claim 14, or a kit according to any of claims 19 to 21 for the preparation of a medicament for the treatment or amelioration of a disease or disorder substantially caused by missing or defective expression of a protein including anemia (erythropoietin), diabetes (insulin), hemophilia (coagulation factors VIII or IX), lactose intolerance (beta-lactamase), adenosine deaminase deficiency (adenosine deaminase), or diseases or disorders include diseases or disorders caused by degenerative cell proliferation or aberrant cellular activity, including cancer, allergy, autoimmune diseases, or infectious diseases, or diseases caused by bacterial, viral, fungal or parasite pathogens.


SEQUENCE LISTING

<110>  Chemotherapeutisches Forschungsinstitut Georg-Speyer-Haus

<120>  Novel expression vector(s) for enhanced expression of a protein
       of interest in eukaryotic or prokaryotic host cells

<130>  GS02P001EP

<160>  21

<170>  PatentIn version 3.3

<210>  1
<211>  4
<212>  PRT
<213>  Artificial

<220>
<223>  Description of sequence: Furin consensus recognition sequence
       (Arg-X-X-Arg)


<220>
<221>  misc_feature
<222>  (2)..(3)
<223>  x = any amino acid or other

<400>  1

Arg Xaa Xaa Arg
1


<210>  2
<211>  4
<212>  PRT
<213>  Artificial

<220>
<223>  Description of sequence: exemplary Furin recognition sequence
       (Arg-X-Lys/Arg-Arg)


<220>
<221>  misc_feature
<222>  (2)..(2)
<223>  x = any amino acid or other

<220>
<221>  misc_feature
<222>  (3)..(3)
<223>  x = Lys or Arg

<400>  2

Arg Xaa Xaa Arg
1


<210>  3
<211>  4
<212>  PRT
<213>  Artificial

<220>
<223>  Description of sequence: exemplary Furin recognition sequence
       (Arg-Gln/Tyr-Lys/Arg-Arg)

<220>
<221>  misc_feature
<222>  (2)..(2)
<223>  x = Gln or Tyr

<220>
<221>  misc_feature
<222>  (3)..(3)
<223>  x = Lys or Arg

<400>  3

Arg Xaa Xaa Arg
1


<210>  4
<211>  6
<212>  PRT
<213>  Artificial

<220>
<223>  Description of sequence: Furin recognition sequence "fur"

<400>  4

Arg Ala Arg Tyr Lys Arg
1               5


<210>  5
<211>  7
<212>  PRT
<213>  Artificial

<220>
<223>  Description of sequence: Furin recognition sequence "furS"

<400>  5

Arg Ala Arg Tyr Lys Arg Ser
1               5


<210>  6
<211>  38
<212>  DNA
<213>  Artificial

<220>
<223>  Description of sequence: oligonucleotide primer 5'NdeI MBP

<400>  6
aaaaaaacat atgatgaaaa ctgaagaagg taaactgg                              38


<210>  7
<211>  73
<212>  DNA
<213>  Artificial

<220>
<223>  Description of sequence: oligonucleotide primer 3'XhoI SalI furS
       MBP

<400>  7
tttttctcga gtttttgtcg acgctacgtt tgtaacgagc acggctcgaa ttagtctgcg      60

cgtctttcag ggc                                                          73

<210>  8
<211>  34
<212>  DNA
<213>  Artificial

<220>
<223>  Description of sequence: oligonucleotide primer 5'XbaI furS GrB

<400>  8
atatctagaa cgtgctcgtt acaaacgtag catc                                  34

<210>  9
<211>  36
<212>  DNA
<213>  Artificial

<220>
<223>  Description of sequence: oligonucleotide primer 5'XbaI fur GrB

<400>  9
atatctagaa cgtgctcgtt acaaacgtat catcgg                                36

<210>  10
<211>  33
<212>  DNA
<213>  Artificial

<220>
<223>  Description of sequence: oligonucleotide primer 3'XhoI H

<400>  10
tttgatctcg agtcagtgat ggtgatgatg gtg                                   33

<210>  11
<211>  34
<212>  DNA
<213>  Artificial

<220>
<223>  Description of sequence: oligonucleotide primer 5'NdeI MBP/3'XbaI
       MBP

<400>  11
aaatctagag ccgagctcga attagtcgcg cgtc                                  34

<210>  12
<211>  46
<212>  DNA
<213>  Artificial

<220>
<223>  Description of sequence: oligonucletide primer 5'NdeI GST

<400>  12
aaaaaaacat atgatgtccc ctatactagg ttattggaaa attaag                     46

<210>  13
<211>  48
<212>  DNA

<213>  Artificial

<220>
<223>  Description of sequence: oligonucletide primer 3'XbaI GST

<400>  13
aaatctagag catccgattt tggaggatgg tcgccaccac caaacgtg                48

<210>  14
<211>  24
<212>  DNA
<213>  Artificial

<220>
<223>  Description of sequence: oligonucleotide primer GAPDH Pichia
       forward

<400>  14
ggctatcact gtcggtatta acgg                                          24

<210>  15
<211>  24
<212>  DNA
<213>  Artificial

<220>
<223>  Description of sequence: oligonucleotide primer GAPDH Pichia
       reverse

<400>  15
gtggagtaac cgtactcgtt gtcg                                          24

<210>  16
<211>  21
<212>  DNA
<213>  Artificial

<220>
<223>  Description of sequence: oligonucleotide primer GrB-forward

<400>  16
tggaggccct cttgtgtgta a                                             21

<210>  17
<211>  19
<212>  DNA
<213>  Artificial

<220>
<223>  Description of sequence: oligonucleotide primer GrB-reverse

<400>  17
gcatgccatt gtttcgtcc                                                19

<210>  18
<211>  23
<212>  DNA
<213>  Artificial

<220>
<223>  Description of sequence:  oligonucleotide primer GrB-so

<400>  18
aaggtggccc agggcattgt ctc                                           23

<210>  19
<211>  22
<212>  DNA
<213>  Artificial

<220>
<223>  Description of sequence:  oligonucleotide primer GADPH-forward

<400>  19
gtcaacgaca ctttcggaat tg                                              22


<210>  20
<211>  18
<212>  DNA
<213>  Artificial

<220>
<223>  Description of sequence:  oligonucleotide primer GADPH-reverse

<400>  20
ttgagtggcg gtcatgga                                                   18


<210>  21
<211>  23
<212>  DNA
<213>  Artificial

<220>
<223>  Description of sequence:  oligonucleotide primer GADPH-so

<400>  21
tccggtttga tgaccaccgt cca                                             23